(19) Europäisches Patentamt
European Patent Office
Office européen des brevets

(11) **EP 4 197 577 B1**

(12) **EUROPÄISCHE PATENTSCHRIFT**

(45) Veröffentlichungstag und Bekanntmachung des Hinweises auf die Patenterteilung:
**27.08.2025 Patentblatt 2025/35**

(21) Anmeldenummer: **22212761.5**

(22) Anmeldetag: **12.12.2022**

(51) Internationale Patentklassifikation (IPC):
*A61N 1/36* (2006.01)   *G16H 40/63* (2018.01)
*A61M 16/00* (2006.01)   *G16H 20/40* (2018.01)
*A61B 5/08* (2006.01)

(52) Gemeinsame Patentklassifikation (CPC):
(C-Sets verfügbar)
**A61M 16/024; A61N 1/3601; A61N 1/36014; G16H 20/40; G16H 40/63;** A61B 5/08; A61M 16/0069; A61M 2016/0018; A61M 2016/0027; A61M 2016/0036; A61M 2205/502; A61M 2205/58; A61M 2230/04; A61M 2230/60     (Forts.)

(54) **VERFAHREN UND VORRICHTUNG ZUR ÜBERWACHUNG EINES BEATMUNGSGERÄTS**

METHOD AND DEVICE FOR MONITORING A VENTILATOR

PROCÉDÉ ET DISPOSITIF DE SURVEILLANCE D'UN APPAREIL RESPIRATOIRE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **16.12.2021 DE 102021133485**

(43) Veröffentlichungstag der Anmeldung:
**21.06.2023 Patentblatt 2023/25**

(73) Patentinhaber: **Drägerwerk AG & Co. KGaA
23558 Lübeck (DE)**

(72) Erfinder:
• **Handzsuj, Thomas
23558 Lübeck (DE)**
• **Eger, Marcus
23558 Lübeck (DE)**

(56) Entgegenhaltungen:
**EP-B1- 3 274 031     WO-A1-2013/071404**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

   C-Sets
   A61M 2230/04, A61M 2230/005;
   A61M 2230/60, A61M 2230/005

## Beschreibung

Die Offenbarung betrifft ein Überwachungs-Verfahren und eine Überwachungs-Vorrichtung, um ein Beatmungsgerät ("ventilator") automatisch zu überwachen.

Ein Beatmungsgerät vermag einen Patienten künstlich zu beatmen. Der Patient ist in der Regel mit einer patientenseitigen Koppeleinheit, beispielsweise mit einer Atemmaske auf seinem Gesicht oder einem Tubus oder Katheter in seinem Körper, verbunden. Das Beatmungsgerät steht wenigstens zeitweise in einer Fluidverbindung mit der patientenseitigen Koppeleinheit und führt eine Abfolge von Beatmungshüben aus. Bei jedem Beatmungshub wird eine Menge eines Gasgemischs, welches Sauerstoff umfasst, zu der patientenseitigen Koppeleinheit und weiter in den Körper des Patienten gefördert.

In einer ersten Art der künstlichen Beatmung umfasst das Gasgemisch mindestens ein Narkosemittel, und der Patient ist vollständig narkotisiert. In einer zweiten Art unterstützt das Beatmungsgerät die eigene Atmungsaktivität des Patienten. Die eigene Atmungsaktivität wird von der Atmungsmuskulatur des Patienten ausgeübt. Seine eigene Atmungsmuskulatur bewirkt, dass der Patient Atemluft ansaugt. Elektrische Signale, die im Körper des Patienten erzeugt werden, regen die eigene Atmungsmuskulatur an. Möglich ist auch, dass die eigene Atmungsmuskulatur von einem entsprechenden Gerät extern stimuliert wird.

Die Offenbarung bezieht sich auf eine unterstützende künstliche Beatmung. Auch bei dieser unterstützenden künstlichen Beatmung kann dem Patienten ein Narkosemittel zugeführt werden.

Gewünscht wird, dass die Beatmungshübe, welche das Beatmungsgerät bei der unterstützenden künstlichen Beatmung ausführt, gut mit der eigenen Atmungsaktivität des Patienten synchronisiert sind. Eine ideale Synchronisation liegt vor, wenn ein Beatmungshub genau dann beginnt, wenn die eigene Atmungsmuskulatur des Patienten einen Atemzug beginnt oder wenigstens versucht, und der Beatmungshub genau dann endet, wenn die eigene Atmungsmuskulatur den Atemzug oder den Versuch des Atemzugs beendet. Idealerweise detektiert das Beatmungsgerät jeden ausgeführten oder versuchten Atemzug des Patienten und reagiert auf jeden Atemzug mit einem Beatmungshub, führt aber einen Beatmungshub nur als Reaktion auf einen detektierten Atemzug aus.

Eine ideale Synchronisation lässt sich in aller Regel in der Praxis nicht erzielen. In US 9,392,964 B2 und WO 2013 / 071 404 A1 werden verschiedene Arten von Asynchronien zwischen der eigenen Atmungsaktivität des Patienten und einer künstlichen Beatmung beschrieben. Verschiedene Merkmale werden vorgestellt, um solche Asynchronien zu erkennen und um die künstliche Beatmung durch das Beatmungsgerät bei Bedarf zu verändern.

Der Erfindung liegt die Aufgabe zugrunde, eine Überwachungs-Vorrichtung bereitzustellen, welche ein Beatmungsgerät während der unterstützenden künstlichen Beatmung daraufhin überwacht, wie gut die Beatmungshübe des Beatmungsgeräts mit der eigenen Atmungsaktivität des Patienten synchronisiert sind.

Die Aufgabe wird durch eine Beatmungs-Anordnung mit den Merkmalen des Anspruchs 1 gelöst.

Vorteilhafte Ausgestaltungen sind in den Unteransprüchen angegeben.

Die offenbarungsgemäße signalverarbeitende Überwachungseinheit vermögen automatisch ein Beatmungsgerät zu überwachen.

Das überwachte Beatmungsgerät vermag eine unterstützende künstliche Beatmung eines Patienten durchzuführen. Bei der unterstützenden künstlichen Beatmung führt das Beatmungsgerät eine Abfolge von Beatmungshüben aus, und der Patient unternimmt eine Abfolge von Einatmungs-Anstrengungen. Durch jeden Beatmungshub wird jeweils eine Menge eines Gasgemischs umfassend Sauerstoff zu dem Patienten gefördert. In einer Ausgestaltung enthält dieses Gasgemisch zusätzlich ein Narkosemittel, sodass der Patient teilweise oder vollständig sediert, aber nicht vollständig narkotisiert ist. Möglich ist auch, dass das Gasgemisch frei von einem Narkosemittel ist. Der Anteil von Sauerstoff im Gasgemisch kann höher als der Anteil von Sauerstoff in der Atemluft sein.

Die Atmungsmuskulatur des Patienten führt eine eigene Atmungsaktivität aus, während der Patient vom Beatmungsgerät künstlich beatmet wird. Die eigene Atmungsaktivität des Patienten umfasst insbesondere seine spontane Atmung, welche durch elektrische Impulse ausgelöst wird, die im Körper des Patienten erzeugt werden. Die Atmungsmuskulatur des Patienten kann stattdessen oder zusätzlich durch ein geeignetes Gerät von außen stimuliert werden, beispielsweise durch elektrische Signale oder in einem Magnetfeld. Die spontane Atmung und die Stimulation von außen können sich überlagern.

Idealerweise führt die eigene Atmungsaktivität des Patienten dazu, dass der Patient Atemluft oder ein sonstiges Gasgemisch mit Sauerstoff einatmet und wieder ausatmet. Möglich ist aber, dass zwar der Patient versucht, einzuatmen, es ihm jedoch nicht gelingt, eine relevante Menge des Gasgemischs einzuatmen. Beispielsweise ist die Lunge nicht elastisch genug, und nach einem Ausatmen verbleibt noch eine relativ große Menge von verbrauchter Luft in der Lunge. Oder die Atmungsmuskulatur des Patienten ist geschwächt. Daher führt das Beatmungsgerät eine Abfolge von Beatmungshüben mit dem Ziel aus, dass jede Einatmungs-Anstrengung des Patienten einen Beatmungshub auslöst und nicht nur jeder tatsächlich durchgeführte Einatmungs-Vorgang.

Unter einer einzelnen Einatmungs-Anstrengung wird ein Versuch des Patienten verstanden, Gas in die Lunge einzusaugen. Eine Einatmungs-Anstrengung kann gelingen, also zu einem Einatmungs-Vorgang führen, bei dem eine relevante Menge des Gases in die Lunge des Patienten fließt, sodass ein Einatmungs-Vorgang ein Sonderfall einer Einatmungs-Anstrengung ist. Es kann auch beim Versuch bleiben. Idealerweise führen also sowohl ein vollendeter als auch ein nur versuchter Einatmungs-Vorgang zu jeweils einem Beatmungshub des Beatmungsgeräts.

Das Beatmungsgerät empfängt Messwerte von mindestens einem Atmungs-Sensor, optional von mehreren Atmungs-Sensoren. Der oder mindestens ein Atmungs-Sensor, dessen Messwerte an das Beatmungsgerät übertragen werden, werden, bevorzugt jeder verbundene Atmungs-Sensor, vermag jeweils eine Größe zu messen, die mit den eigenen Einatmungs-Anstrengungen des Patienten korreliert. Dieser Atmungs-Sensor vermag also idealerweise nicht nur einen Einatmungs-Vorgang zu erfassen, bei dem eine relevante Menge von Gas in die Lunge fließt, sondern auch eine Einatmungs-Anstrengung, die nicht zum Einatmen einer relevanten Gasmenge führt. Die gemessene Größe kann insbesondere eine pneumatische oder elektrische oder mechanische oder optische Größe sein. Der oder mindestens ein Atmungs-Sensor kann ein Bestandteil des Beatmungsgeräts sein. Möglich ist auch, dass der oder ein Atmungs-Sensor mindestens einen Messwertaufnehmer im Körper oder in der Nähe des Körpers des Patienten umfasst.

Das Beatmungsgerät generiert mindestens ein respiratorisches Signal. Um das Signal zu generieren, verwendet und verarbeitet das Beatmungsgerät empfangene Messwerte des oder mindestens eines Atmungs-Sensors. Das oder jedes generierte respiratorische Signal ist jeweils ein Maß für die eigene Atmungsaktivität - genauer gesagt: für die eigenen Einatmungs-Anstrengungen - des Patienten. Insbesondere korreliert das respiratorische Signal mit dem Volumenfluss von Gas zum und optional vom Patienten.

Möglich ist, dass das Beatmungsgerät mehrere respiratorische Signale generiert, wobei jedes generierte respiratorische Signal jeweils ein Maß für die eigene Atmungsaktivität desselben Patienten ist und wobei das Beatmungsgerät bevorzugt die Messwerte von verschiedenen Atmungs-Sensoren verwendet, um die unterschiedlichen respiratorischen Signale zu generieren. Idealerweise stimmen die respiratorischen Signale miteinander überein, in der Praxis differieren sie in der Regel voneinander.

Jede Einatmungs-Anstrengung des Patienten hat einen Beginn und ein Ende. Das Beatmungsgerät detektiert in dem oder mindestens einem respiratorischen Signal oder in einer Zusammenfassung von mehreren respiratorischen Signalen den jeweiligen Beginn und das jeweilige Ende jeder Einatmungs-Anstrengung und damit insbesondere den Beginn und das Ende jedes erfolgreichen Einatmungs-Vorgangs. Möglich ist, dass das Beatmungsgerät in mehreren respiratorischen Signalen jeweils einen Beginn und ein Ende derselben Einatmungs-Anstrengung detektiert und aus diesen detektierten Werten durch Aggregation einen Beginn und ein Ende dieser Einatmungs-Anstrengung detektiert.

Das Beatmungsgerät löst die Abfolge von Beatmungshüben mit dem Ziel aus, dass jede Einatmungs-Anstrengung des Patienten genau einen Beatmungshub des Beatmungsgeräts auslöst und umgekehrt jeder Beatmungshub von genau einer Einatmungs-Anstrengung ausgelöst wird. Idealerweise stimmen der Beginn und das Ende der Einatmungs-Anstrengung mit dem Beginn und dem Ende genau eines Beatmungshubs überein, und der Beatmungshub wird während der gesamten Einatmungs-Anstrengung ununterbrochen durchgeführt. In der Praxis lässt sich diese ideale Synchronisation in der Regel nicht erzielen. Vielmehr treten Asynchronien auf.

In einer bevorzugten Ausgestaltung umfasst das Beatmungsgerät eine Signalverarbeitungseinheit. Diese Signalverarbeitungseinheit des Beatmungsgeräts empfängt Messwerte von dem oder mindestens einem Atmungs-Sensor, generiert abhängig von diesen Messwerten das oder mindestens ein respiratorisches Signal und löst abhängig von dem oder einem respiratorischen Signal die Abfolge von Beatmungshüben aus - genauer gesagt: den Beginn und das Ende jedes Beatmungshubs. In einer Ausgestaltung ist die offenbarungsgemäße Überwachungseinheit ein Bestandteil des Beatmungsgeräts. Möglich ist, dass die Signalverarbeitungseinheit, welche die Abfolge von Beatmungshüben auslöst, und die Überwachungseinheit auf demselben signalverarbeitenden Gerät oder auf zwei verschiedenen Geräten des Beatmungsgeräts realisiert sind.

Erfindungsgemäß sind mindestens zwei mögliche Asynchronie-Art vorgegeben, die während der unterstützenden künstlichen Beatmung des Patienten durch das Beatmungsgerät auftreten können. Bevorzugt sind mehr als zwei verschiedene mögliche Asynchronie-Arten vorgegeben. Vorgegeben ist jeweils ein Kriterium, wann eine vorgegebene Asynchronie-Art tatsächlich aufgetreten ist. Dieses Kriterium hängt von den eigenen Einatmungs-Anstrengungen des Patienten sowie von der Abfolge von Beatmungshüben des Beatmungsgeräts ab.

Eine vorgegebene mögliche Asynchronie-Art ist tatsächlich aufgetreten, wenn die tatsächliche künstliche Beatmung von einer ideal synchronisierten künstlichen Beatmung abweicht und wenn bei der künstlichen Beatmung eines der folgenden Ereignisse eingetreten ist:

- Der Beatmungshub beginnt oder endet früher als die auslösende Einatmungs-Anstrengung.
- Der Beatmungshub beginnt oder endet später als die auslösende Einatmungs-Anstrengung.
- Ein Beatmungshub wird ohne eine Einatmungs-Anstrengung ausgelöst, d.h. der Beatmungshub wird fälschlicherweise ausgelöst.
- Eine Einatmungs-Anstrengung löst keinen Beatmungshub aus, d.h. die Einatmungs-Anstrengung wird nicht detektiert.

Zwei Sonderfälle für Asynchronie-Arten sind, dass

- ein Beatmungshub fälschlich unterbrochen und anschließend ein neuer Beatmungshub begonnen wird, während der Patient eine einzige Einatmungs-Anstrengung durchführt, und
- dass ein Beatmungshub fälschlich fortgesetzt wird, während der Patient eine Einatmungs-Anstrengung beendet und anschließend eine neue Einatmungs-Anstrengung beginnt.

Die Überwachungseinheit ist dazu ausgestaltet, automatisch jedes Auftreten jeder vorgegebenen möglichen Asynchronie-Art zu detektieren, also einerseits zu detektieren, dass eine Asynchronie aufgetreten ist, und außerdem zu detektieren, von welcher Asynchronie-Art diese Asynchronie ist. Falls die unterstützende künstliche Beatmung ideal mit den eigenen Einatmungs-Anstrengungen des Patienten synchronisiert ist, so tritt keine dieser möglichen Asynchronie-Arten tatsächlich auf. Eine ideal synchronisierte künstliche Beatmung lässt sich in der Regel aber nicht erzielen. Das offenbarungsgemäße Überwachungs-Verfahren detektiert automatisch jedes Auftreten einer vorgegebenen möglichen Asynchronie-Art, und zwar zumindest dann, wenn die Dauer, während der diese Asynchronie-Art tatsächlich auftritt, länger als eine vorgegebene Zeitdauer-Schranke ist. Natürlich ist möglich, dass dieselbe Asynchronie-Art mehrmals hintereinander auftritt, und zwar häufig mit unterschiedlichen Dauern.

Offenbarungsgemäß ermitteln die Überwachungseinheit und das Überwachungs-Verfahren für jede vorgegebene mögliche Asynchronie-Art jeweils ein Maß dafür, wie oft und / oder wie lange diese Asynchronie-Art tatsächlich aufgetreten ist, während das Beatmungsgerät die Abfolge von Beatmungshüben durchführt, ermitteln also ein Maß für die Häufigkeit und / oder ein Maß für die Dauer. Möglich ist, dass ein Maß für die Häufigkeit und zusätzlich ein Maß für die Dauer ermittelt wird.

Erfindungsgemäß wird für mindestens zwei verschiedene Asynchronie-Arten jeweils ein Maß für die Häufigkeit und / oder die Dauer ermittelt. Erfindungsgemäß werden also mindestens zwei Maße für zwei unterschiedliche Arten von Asynchronien ermittelt. Dieses Merkmal ermöglicht eine gezieltere Überwachung und Anpassung des Beatmungsgeräts, als wenn lediglich pauschal festgestellt werden würde, wie oft und / oder wie lange und / oder wie viele Beatmungshübe des Beatmungsgeräts nicht ideal mit der eigenen Atmungsaktivität des Patienten synchronisiert sind. Insbesondere wird erleichtert, das Beatmungsgerät an die Erfordernisse für die Beatmung eines bestimmten Patienten anzupassen. Dieser Anpassung kann manuell oder auch automatisch durchgeführt werden.

Offenbarungsgemäß detektieren die Überwachungseinheit und das Überwachungs-Verfahren ein Auftreten einer möglichen Asynchronie-Art mindestens dann, wenn dieses Auftreten länger dauert als eine vorgegebene Zeitdauer-Schranke, bevorzugt nur dann. Die vorgegebene Zeitdauer-Schranke kann für alle Asynchronie-Arten dieselbe sein oder für mindestens zwei verschiedene Asynchronie-Arten unterschiedlich sein. Durch die Berücksichtigung der oder jeder Zeitdauer-Schranke wird eine nur sehr kurz auftretende Asynchronie nicht als relevant gewertet und wird daher nicht berücksichtigt. Ein solches nur kurzes Auftreten ist in der Regel für den Patienten unschädlich.

Möglich ist, dass ein Standardwert für die Zeitdauer-Schranken vorgegeben wird und ein Benutzer einen abweichenden Wert für eine Zeitdauer-Schranke vorgeben kann. Die oder jede Zeitdauer-Schranke für eine Asynchronie-Art kann fest vorgegeben sein oder von mindestens einem gemessenen oder geschätzten Vitalparameter des Patienten abhängen, beispielsweise von der Lungenzeitkonstante des Patienten, und / oder von mindestens einem Parameter der künstlichen Beatmung, beispielsweise dem Volumen eines Totraums in der Fluidverbindung zwischen dem Beatmungsgerät und der Lunge des Patienten. Möglich ist, dass die Überwachungseinheit die oder wenigstens eine Zeitdauer-Schranke abhängig von einem Wert eines Vitalparameter des Patienten berechnet.

Das ermittelte Maß dafür, wie oft und / oder wie lange eine Asynchronie-Art tatsächlich aufgetreten ist, lässt sich dafür verwenden, um das Beatmungsgerät noch besser an die eigene Atmungsaktivität des künstlich beatmeten Patienten anzupassen und es hierbei mit dessen eigenen Einatmungs-Anstrengungen zu synchronisieren. Dies wird nachfolgend näher erläutert.

In der Regel hat das oder jedes respiratorische Signal einen oszillierenden Verlauf und korreliert insbesondere mit dem Volumenfluss vom Beatmungsgerät zum Patienten und optional zurück vom Patienten zum Beatmungsgerät. Um die Beatmungshübe synchronisiert mit den Einatmungs-Anstrengungen des Patienten auszulösen, ist es erforderlich, dass das Beatmungsgerät in dem oder mindestens einem respiratorischen Signal den jeweiligen Beginn und das jeweilige Ende jeder Einatmungs-Anstrengung detektiert.

Um in dem oder wenigstens einem respiratorischen Signal den Beginn und das Ende einer Einatmungs-Anstrengung zu detektieren, wendet das Beatmungsgerät in der Regel eine vorgegebene rechnerauswertbare Entscheidungsvorschrift an. Diese Entscheidungsvorschrift hängt von mindestens einem Parameter ab, beispielsweise von einer unteren Schranke für die Menge oder die Dauer des Volumenflusses. Die Kenntnis, wie oft und / oder wie lange jede der vorgegebenen möglichen Asynchronie-Arten jeweils aufgetreten ist, erleichtert es, dem oder jedem Parameter für die EntscheidungsVorschrift einen angepassten Wert zuzuordnen und dadurch die unterstützende künstliche Beatmung noch besser an die eigene Atmungsaktivität - genau: an die eigenen Einatmungs-Anstrengungen - des Patienten anzupassen.

Gemäß der Ausgestaltung berechnet die Überwachungseinheit einen Sollwert für den oder einen Parameter der Entscheidungsvorschrift. Um diesen Sollwert zu berechnen, verwendet die Überwachungseinheit mindestens ein ermitteltes Maß für die Häufigkeit und / oder Dauer einer möglichen Asynchronie-Art. Beispielsweise verwendet die Überwachungseinheit das ermittelte Maß für diejenige Asynchronie-Art, die in einer vorgegebenen Zeitspanne oder seit Beginn der unterstützenden künstlichen Beatmung tatsächlich am häufigsten und / oder am längsten aufgetreten ist. Möglich ist, dass die Überwachungseinheit den Sollwert abhängig von dem jeweils ermittelten Maß von mindestens zwei verschiedenen Asynchronie-Arten berechnet.

Die Überwachungseinheit erzeugt eine Nachricht. In einer Realisierungsform umfasst diese Nachricht eine Information über den berechneten Sollwert. In einer anderen Realisierungsform umfasst diese Nachricht eine Information über eine gewünschte oder erforderliche oder bereits durchgeführte Veränderung des aktuell verwendeten Werts des Parameters, wobei diese Veränderung vom berechneten Sollwert abhängt. Diese beiden Realisierungsformen lassen sich miteinander kombinieren.

In einer Realisierungsform bewirkt die Überwachungseinheit, dass die Nachricht an das Beatmungsgerät übermittelt wird. Das Beatmungsgerät verwendet diese Nachricht, um bei Bedarf den Wert des Parameters der Entscheidungsvorschrift automatisch anzupassen. In einer anderen Realisierungsform bewirkt die Überwachungseinheit, dass die Nachricht in einer von einem Menschen wahrnehmbaren Form ausgegeben wird. Das Beatmungsgerät erfasst eine Benutzereingabe und verändert den Wert für den Parameter der Entscheidungsvorschrift abhängig von der Benutzereingabe. Dadurch kann ein Benutzer überprüfen, ob dem Parameter tatsächlich ein anderer Wert zugewiesen werden soll oder nicht, und die vorgeschlagene Veränderung bestätigen oder verwerfen. Indem abhängig von der Nachricht dem Parameter der Entscheidungsvorschrift ein anderer Wert zugewiesen wird, wird in vielen Fällen erreicht, dass mindestens eine Asynchronie-Art seltener und / oder kürzer auftritt.

In vielen Fällen ermöglicht die Offenbarung es, dass diese Anpassung der unterstützenden künstlichen Beatmung schneller und / oder gezielter durchgeführt wird als bei anderen möglichen Vorgehensweisen. Denn erfindungsgemäß wird für mindestens zwei verschiedene mögliche Asynchronie-Arten das jeweilige Maß für die Dauer und / oder die Häufigkeit ermittelt. Dies ermöglicht ein gezielteres Vorgehen, als wenn nur allgemein detektiert werden würde, wie oft und / oder wie lange die Beatmungshübe des Beatmungsgeräts nicht mit der eigenen Atmungsaktivität des Patienten synchronisiert sind.

In manchen Fällen lässt sich ein Ergebnis der Überwachungseinheit auch dafür verwenden, um einen Fehler eines Atmungs-Sensors zu detektieren. Beispielsweise ist der Atmungs-Sensor nicht korrekt positioniert oder nicht korrekt am Körper des Patienten befestigt oder defekt. Oder Messwerte dieses Atmungs-Sensors werden nicht korrekt an das Beatmungsgerät übermittelt. Ein solcher Fehler eines Sensors führt häufig dazu, dass eine bestimmte Asynchronie-Art häufiger und / oder länger auftritt als bei einem korrekt positionierten und arbeitenden Sensor. Ein erfindungsgemäß ermitteltes Maß für diese Asynchronie-Art kann also ein Hinweis auf einen falsch positionierten oder befestigten oder defekten Atmungs-Sensor sein.

In einer Ausgestaltung wird eine Abfolge von Abtast-Zeitpunkten vorgegeben. Die Überwachungseinheit ermittelt für jeden vorgegebenen Abtast-Zeitpunkt jeweils, welche der folgenden vier möglichen Situationen an diesem Abtast-Zeitpunkt vorliegt:

- Der Patient führt eine Einatmungs-Anstrengung aus. Das Beatmungsgerät führt einen Beatmungshub aus.
- Der Patient führt keine Einatmungs-Anstrengung aus. Das Beatmungsgerät führt keinen Beatmungshub aus.
- Der Patient führt eine Einatmungs-Anstrengung aus. Das Beatmungsgerät führt keinen Beatmungshub aus.
- Das Beatmungsgerät führt einen Beatmungshub aus. Der Patient führt keine Einatmungs-Anstrengung aus.

An jedem Abtast-Zeitpunkt während der unterstützenden künstlichen Beatmung liegt genau eine der vier möglichen Situationen vor. Bei einer ideal synchronisierten unterstützenden künstlichen Beatmung treten zu jedem Abtast-Zeitpunkt nur die ersten beiden Situationen auf. Die beiden letzten Situationen sind ein Indiz für das Auftreten einer Asynchronie.

Um zu ermitteln, welche dieser vier möglichen Situationen an einem Abtast-Zeitpunkt tatsächlich vorliegt, wertet die Überwachungseinheit das oder mindestens ein respiratorisches Signal aus und ermittelt außerdem, wann ein Beatmungshub beginnt und wann er endet. Um zu ermitteln, wann ein Beatmungshub beginnt und wann er endet, verwendet die Überwachungseinheit bevorzugt ein Signal einer Steuerung des Beatmungsgeräts und optional mindestens einen Parameter einer Fluidverbindung zwischen dem Beatmungsgerät und dem Patienten. Möglich ist auch, laufend den Volumenfluss in einer Fluidverbindung vom Beatmungsgerät zum Patienten zu messen.

Zu jedem Abtast-Zeitpunkt wird genau eine der oben genannten vier möglichen Situationen detektiert. Weil diese Detektion für eine Abfolge von Abtast-Zeitpunkten durchgeführt wird, liegt eine Abfolge von Situationen vor, nämlich pro Abtast-Zeitpunkt die jeweils detektierte Situation. Die Überwachungseinheit detektiert in dieser Situationen-Abfolge jede Situationen-Sequenz. Eine zu detektierende Situationen-Sequenz hat folgende Eigenschaften:

- Die Situationen-Sequenz besteht aus mindestens zwei unmittelbar aufeinanderfolgenden Situationen, bevorzugt aus mindestens drei unmittelbar aufeinanderfolgenden Situationen, besonders bevorzugt aus genau drei unmittelbar aufeinanderfolgenden Situationen.
- Die beiden oder jeweils zwei unmittelbar aufeinanderfolgende Situationen der Situationen-Sequenz unterscheiden sich voneinander.

Möglich ist, dass eine Situationen-Sequenz aus einer ersten Situation, aus einer nachfolgenden zweiten Situation und nachfolgend wieder aus der ersten Situation besteht. Eine Situationen-Sequenz kann das Auftreten einer Asynchronie bedeuten oder auch einen ideal synchronisierten Beatmungshub ohne Asynchronie.

Erfindungsgemäß ermittelt die Überwachungseinheit für jede vorgegebene mögliche Asynchronie-Art jeweils ein Maß für die Häufigkeit und / oder die Dauer, mit der diese Asynchronie-Art tatsächlich aufgetreten ist. Gemäß der gerade beschriebenen Ausgestaltung ermittelt die Überwachungseinheit für jede vorgegebene mögliche Asynchronie-Art, welche detektierte Situationen-Sequenzen bedeuten, dass diese Asynchronie-Art tatsächlich aufgetreten ist. Selbstverständlich ist es möglich, dass für mindestens eine mögliche Asynchronie-Art keine passende Situationen-Sequenz detektiert wird oder aber mehrere zeitlich voneinander beabstandete passende Situationen-Sequenzen detektiert werden. Um das Maß für die Häufigkeit und / oder die Dauer einer möglichen Asynchronie-Art zu ermitteln, verwendet die Überwachungseinheit die passenden Situationen-Sequenzen.

Diese Ausgestaltung ermöglicht es, rasch zwei verschiedene Signale miteinander zu vergleichen, nämlich das oder mindestens ein respiratorisches Signal und ein Signal, welches die Abfolge der Beatmungshübe beschreibt. Indem die vier möglichen Situationen ermittelt werden, wird von quantitativen Parametern der künstlichen Beatmung abstrahiert, insbesondere von der Stärke eines Volumenflusses vom Beatmungsgerät zum Patienten und / oder der Stärke einer eigenen Einatmungs-Anstrengung des Patienten. Diese Ausgestaltung erhöht in vielen Fällen die Zuverlässigkeit der Ermittlung und / oder spart Rechenzeit und / oder Rechenkapazität ein.

in einer Realisierungsform dieser Ausgestaltung ermittelt die Überwachungseinheit für jede mögliche Asynchronie-Art, wie häufig passende Situationen-Sequenzen auftreten und / oder wie lange die passenden Situationen-Sequenzen insgesamt andauern. Unter Verwendung dieser Häufigkeit und / oder Dauer ermittelt die Überwachungseinheit das Maß für die Häufigkeit und / oder Dauer der Asynchronie-Art.

Jede der vorgegebenen möglichen Asynchronie-Arten beschreibt jeweils eine mögliche Asynchronie zwischen den Einatmungs-Anstrengungen des Patienten und der unterstützenden künstlichen Beatmung. Bevorzugt werden acht mögliche Asynchronie-Arten vorgegeben, nämlich vier Zeit-Asynchronien und vier Ereignis-Asynchronien. Für jede dieser acht möglichen Asynchronie-Arten wird jeweils ein Maß für deren Häufigkeit und / oder Dauer ermittelt.

Die folgenden vier möglichen Asynchronie-Arten sind Zeit-Asynchronien, d.h. ein Beatmungshub wird zwar abhängig von einer Einatmungs-Anstrengung ausgelöst, aber zu früh oder zu spät:

- Ein Beatmungshub löst eine Einatmungs-Anstrengung aus (Reverse Triggering).
- Ein Beatmungshub wird zu spät ausgelöst (Late Triggering).
- Ein Beatmungshub wird zu spät beendet (Late Cycling Off).
- Ein Beatmungshub wird zu früh beendet (Premature Cycling Off).

Die übrigen vier möglichen Asynchronie-Arten sind Ereignis-Asynchronien, d.h. ein Beatmungshub wird fälschlicherweise ausgelöst (keine Einatmungs-Anstrengung) oder fälschlicherweise nicht ausgelöst (Einatmungs-Anstrengung nicht detektiert):

- Ein Beatmungshub wird ohne eine Einatmungs-Anstrengung ausgelöst (Auto Triggering).
- Eine Einatmungs-Anstrengung löst keinen Beatmungshub aus (Missed Effort).
- Eine erste Einatmungs-Anstrengung wird während eines Beatmungshubs beendet, und eine zweite Einatmungs-Anstrengung wird während desselben Beatmungshubs begonnen (Missed Expiration).
- Ein erster Beatmungshub wird während eine Einatmungs-Anstrengung beendet, und ein zweiter Beatmungshub wird während derselben Einatmungs-Anstrengung begonnen (Double Triggering).

Erfindungsgemäß wird mindestens eine Darstellung erzeugt und in einer von einem Menschen wahrnehmbaren Form ausgegeben, beispielsweise visuell auf einer Anzeigeeinheit. Die Überwachungseinheit erzeugt automatisch die oder jede dieser Darstellungen und bewirkt, dass die Anzeigeeinheit die oder jede Darstellung in einer von einem Menschen visuell wahrnehmbaren Form ausgibt. Bevorzugt wird die oder mindestens eine Darstellung laufend aktualisiert, während das Beatmungsgerät eine Abfolge von Beatmungshüben durchführt. Die oder mindestens eine Darstellung bezieht sich beispielsweise auf den gesamten Verlauf der bisherigen künstlichen Beatmung oder auf den Verlauf seit der letzten Kalibrierung des Beatmungsgeräts oder nachdem zum letzten Mal ein Parameterwert des Beatmungsgeräts verändert wurde oder aber auf eine gleitende Zeitspanne vor dem aktuellen Zeitpunkt.

Die oder jede erzeugte Darstellung zeigt auf einen Blick, wie oft und / oder wie lange mindestens zwei der vorgegebenen möglichen Asynchronie-Arten jeweils aufgetreten sind. Bevorzugt ist die Darstellung zusätzlich mit jeweils einer textlichen Beschreibung der möglichen Asynchronie-Art versehen, für die ein Maß dargestellt wird.

Diese Darstellung ermöglicht es einem Benutzer, rasch zu erfassen, nämlich quasi auf einen Blick, wie gut die Beatmungshübe des Beatmungsgeräts mit den Einatmungs-Anstrengungen des Patienten synchronisiert sind. Diese Wirkung wird in vielen Fällen auch dann erzielt, wenn die verwendete oder verwendbare Darstellungsfläche der Anzeigeeinheit relativ klein und / oder nicht optimal beleuchtet und / oder schräg zu einem Betrachter positioniert ist. Die Darstellung erleichtert es einem Benutzer zu entscheiden, ob die unterstützende künstliche Beatmung ausreichend mit den eigenen Einatmungs-Anstrengungen des Patienten synchronisiert ist oder nicht. Weiterhin wird es dem Benutzer erleichtert, bei einer nicht ausreichenden Synchronisierung einem Parameter der künstlichen Beatmung einen Wert zuzuweisen, der für diesen Patienten besser geeignet ist als der aktuell verwendete Wert.

In einer Realisierungsform erzeugt die Überwachungseinheit für mindestens zwei Asynchronie-Arten, bevorzugt für jede vorgegebene Asynchronie-Art, jeweils sowohl ein Maß für die Häufigkeit als auch ein Maß für die Dauer dieser Asynchronie-Art. Bevorzugt werden in der gerade beschriebenen Darstellung beide Maße für die mindestens zwei Asynchronie-Arten dargestellt, insgesamt also mindestens vier Maße. Diese Darstellung erleichtert es einem Benutzer in vielen Fällen noch besser zu beurteilen, wie gut die Beatmungshübe des Beatmungsgeräts mit den Einatmungs-Anstrengungen des Patienten synchronisiert sind. Insbesondere kann ein Benutzer anhand der Darstellung mit den mindestens zwei Asynchronie-Arten und den beiden Maßen pro Asynchronie-Art zwischen verschiedenen möglichen Abhilfen auswählen, um Hinweise zu erhalten, wie sich die Synchronisierung verbessern lässt, oder kann die Synchronisierung verbessern. Insbesondere kann ein Benutzer in einer Situation abwägen, in der sich eine Asynchronie-Art beseitigen lässt, indem einem Parameter ein größerer Wert zugewiesen wird, und eine andere Asynchronie-Art, indem diesem Parameter ein kleinerer Wert zugewiesen wird.

Die folgende Realisierungsform führt in vielen Fällen zu einer besonders übersichtlichen Darstellung, und zwar auch dann, wenn die Anzeigeeinheit relativ klein und / oder schlecht beleuchtet und / oder schräg positioniert ist. Für jede dargestellte Asynchronie-Art werden gemäß dieser Realisierungsform jeweils zwei Maße ermittelt, nämlich eines für die Häufigkeit und eines für die Dauer. Die erzeugte Darstellung wird unter Verwendung von zwei Achsen dargestellt. Diese beiden Achsen stehen senkrecht oder schräg aufeinander und sind beispielsweise eine x-Achse und eine y-Achse. Auf der einen Achse ist das ermittelte Maß für die Häufigkeit einer Asynchronie-Art aufgetragen, beispielsweise die absolute Anzahl oder die relative Anzahl bezogen auf die Anzahl der Beatmungshübe. Auf der anderen Achse ist das ermittelte Maß für die Dauer der Asynchronie-Art aufgetragen, beispielsweise die absolute Dauer oder die relative Dauer bezogen auf die gesamte Dauer der künstlichen Beatmung, insbesondere seit der letzten Einstellung oder Justierung des Beatmungsgeräts. In der Darstellung wird für jede Asynchronie-Art eine zweidimensionale Fläche gezeigt. Falls die beiden Achsen senkrecht aufeinander stehen, so hat diese Fläche die Form eines Rechtecks. Falls sie schräg aufeinander stehen, so hat diese Fläche die Form eines Trapezes. Die beiden Abmessungen dieser Fläche hängen vom Maß für die Häufigkeit bzw. vom Maß für die Dauer ab. Bevorzugt ist eine Abmessung einer Fläche umso größer, je größer das betreffende Maß ist.

Diese Darstellung zeigt einem Benutzer noch intuitiver an, welche Asynchronie-Arten aktuell relevant sind. Sowohl eine häufig auftretende als auch eine lang andauernde Asynchronie-Art führt zu einer großen Fläche, und dies kann ein Benutzer dank der gerade beschriebenen Realisierungsform rasch erfassen. Die verschiedenen gerade beschriebenen Realisierungsformen der Darstellung führen also zu besonders ergonomischen Ausgestaltungen, um Informationen über den Zustand und die Arbeit des Beatmungsgeräts in einer von einem Menschen wahrnehmbaren Form darzustellen.

Besonders bevorzugt werden zwei Darstellungen erzeugt und laufend aktualisiert, und auf der Anzeigeeinheit wird wahlweise die eine oder die andere Darstellung dargestellt. Beispielsweise kann ein Benutzer zwischen der einen Darstellung und der anderen Darstellung umschalten. Die eine Darstellung zeigt, wie lange und / oder wie oft die vier möglichen Arten von Zeit-Asynchronien aufgetreten sind. Die andere Darstellung zeigt, wie lange und / oder wie oft die vier möglichen Arten von Ereignis-Asynchronien aufgetreten sind. Bevorzugt umfasst jede Darstellung jeweils vier Quadranten, wobei jeder Quadrant für jeweils eine Asynchronie-Art steht. In jedem Quadranten wird jeweils eine Fläche angezeigt, deren Abmessungen von der Dauer und / oder der Häufigkeit der Asynchronie-Art abhängen. Möglich ist auch, beide Darstellungen gleichzeitig anzuzeigen. Dadurch wird ein Benutzer rasch über den aktuellen Zustand des Beatmungsgeräts informiert.

Weiterhin betrifft die Offenbarung ein Computerprogramm. Dieses Computerprogramm lässt sich auf einer signalverarbeitenden Überwachungseinheit ausführen. Diese Überwachungseinheit umfasst einen Prozessor und einen Datenspeicher. Bevorzugt ist das Computerprogramm im Datenspeicher abgespeichert oder lässt sich wenigstens zeitweise dort abspeichern. Der Prozessor vermag das abgespeicherte Computerprogramm auszuführen.

Dauerhaft oder wenigstens zeitweise lässt sich eine Datenverbindung zwischen der Überwachungseinheit und einem Beatmungsgerät herstellen oder ist hergestellt. Dieses Beatmungsgerät vermag eine unterstützende künstliche Beatmung für einen Patienten durchzuführen, wobei der Patient eigene Einatmungs-Anstrengungen ausführt. Die Ausgestaltung eines solchen Beatmungsgeräts wurde weiter oben beschrieben. Über die Datenverbindung lassen sich Daten und / oder Signale vom Beatmungsgerät zur Überwachungseinheit und optional auch Nachrichten von der Überwachungseinheit zum Beatmungsgerät übermitteln. In einer Ausgestaltung vermag die Überwachungseinheit einem Parameter des Beatmungsgeräts automatisch einen Wert zuzuweisen, und das Beatmungsgerät benutzt diesen Parameterwert für die Durchführung der unterstützenden künstlichen Beatmung. In einer Ausgestaltung vermag die Überwachungseinheit eine Anzeigeeinheit des Beatmungsgeräts anzusteuern und zu bewirken, dass auf dieser Anzeigeeinheit eine oben beschriebene Darstellung angezeigt wird. Ein Benutzer kann diese Darstellung wahrnehmen und mit Hilfe einer geeigneten Eingabeeinheit mindestens einem Parameter des Beatmungsgeräts einen anderen Wert zuweisen.

Wenn eine Datenverbindung zwischen dem Beatmungsgerät und der Überwachungseinheit hergestellt ist und die Überwachungseinheit, bevorzugt der Prozessor der Überwachungseinheit, das Computerprogramm ausführt, so bewirkt die Überwachungseinheit, dass ein offenbarungsgemäßes Überwachungs-Verfahren durchgeführt wird.

Die Offenbarung betrifft weiterhin ein Beatmungs-Verfahren zur unterstützenden künstlichen Beatmung eines Patienten durch ein Beatmungsgerät, wobei das Beatmungsgerät eine Abfolge von Beatmungshüben mit dem Ziel ausführt, dass die Beatmungshübe bei der unterstützenden künstlichen Beatmung ideal mit den eigenen Einatmungs-Anstrengungen des Patienten synchronisiert sind. Eine signalverarbeitende Überwachungseinheit überwacht, wie gut die Beatmungshübe mit den eigenen Einatmungs-Anstrengungen des Patienten synchronisiert sind, und ermittelt für jede vorgegebene mögliche Asynchronie-Art jeweils ein Maß dafür, wie oft und / oder wie lange diese Asynchronie-Art tatsächlich aufgetreten ist. Diese Überwachungseinheit ist erfindungsgemäß ausgestaltet. Vorteilhafte Ausgestaltungen des erfindungsgemäßen Überwachungs-Verfahrens sind auch vorteilhafte Ausgestaltungen des Beatmungs-Verfahrens.

Die Offenbarung betrifft weiterhin eine Beatmungs-Anordnung. Die Beatmungs-Anordnung umfasst ein Beatmungsgerät, mindestens einen Atmungs-Sensor und eine offenbarungsgemäße Überwachungseinheit. Der oder jeder Atmungs-Sensor vermag jeweils eine Größe zu messen, die mit den eigenen Einatmungs-Anstrengungen des Patienten korreliert. Das Beatmungsgerät vermag eine unterstützende künstliche Beatmung eines Patienten durchzuführen und ist so wie oben beschrieben ausgestaltet.

In einer Ausgestaltung ist die offenbarungsgemäße Überwachungseinheit ein Bestandteil des Beatmungsgeräts. Diese Ausgestaltung erleichtert die Datenübermittlung zwischen der Überwachungseinheit einerseits und einer internen Steuerung und / oder einer Anzeigeeinheit des Beatmungsgeräts.

Im Folgenden wird die Offenbarung anhand eines Ausführungsbeispiels beschrieben. Hierbei zeigt

Figur 1   beispielhaft einen künstlich beatmeten Patienten, ein Beatmungsgerät sowie mehrere Sensoren;
Figur 2   beispielhaft ein durch Messungen gewonnenes elektrisches Summen-Signal und ein pneumatisches respiratorisches Signal;
Figur 3   schematisch einen beispielhaften Verlauf für eine Abfolge von Atemzügen des Patienten und eine Abfolge von Beatmungshüben des Beatmungsgeräts;
Figur 4   schematisch den zeitlichen Verlauf von vier verschiedenen Situationen während der unterstützenden Beatmung;
Figur 5   eine beispielhafte Darstellung der Häufigkeit von vier Asynchronie-Sequenzen, die Zeit-Asynchronien sind;
Figur 6   eine beispielhafte Darstellung der Häufigkeit von vier Asynchronie-Sequenzen, die Ereignis-Asynchronien sind.

Im Ausführungsbeispiel wird die Offenbarung eingesetzt, um einen Patienten künstlich zu beatmen und dadurch die eigene Atmungsaktivität des Patienten zu unterstützen. Die eigene Atmungsaktivität des Patienten kann durch seine spontane Atmung und / oder durch eine externe Stimulation seiner Atmungsmuskulatur bewirkt werden .

Zwischen dem Atemweg des Patienten und einem Beatmungsgerät ist eine Fluidverbindung hergestellt. Durch diese Fluidverbindung hindurch führt das Beatmungsgerät dem Patienten Atemluft oder ein sonstiges Gasgemisch, welches Sauerstoff enthält, zu. Dieses Gasgemisch kann mindestens ein Narkosemittel enthalten. Die Fluidverbindung kann insbesondere dann, wenn der Patient narkotisiert wird, Bestandteil eines Beatmungskreislaufs zwischen dem Patienten und dem Beatmungsgerät sein.

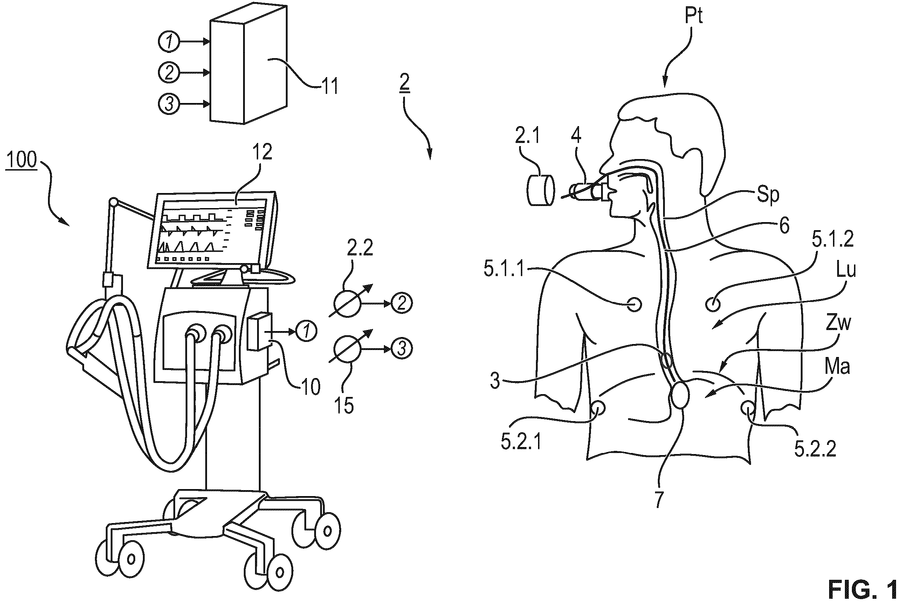

Figur 1 zeigt beispielhaft einen Patienten Pt, der künstlich beatmet wird. Schematisch sind die Lunge Lu, die Speiseröhre Sp, der Magen Ma und das Zwerchfell Zw des Patienten Pt dargestellt. Im Mund des Patienten Pt befindet sich während der künstlichen Beatmung ein flexibles Anschlussstück 4, welches zu einer patientenseitigen Koppeleinheit gehört. In einer Ausgestaltung ist in die Speiseröhre des Patienten Pt ein flexibler Messkatheter 6 gelegt, wobei der Messkatheter 6 in dem Anschlussstück 4 beginnt. Die patientenseitige Koppeleinheit ist wenigstens zeitweise im / oder am Körper des Patienten Pt angebracht und umfasst im Ausführungsbeispiel das Anschlussstück 4 und den Messkatheter 6.

Ein Beatmungsgerät 100 mit einer Anzeige- und Bedieneinheit 12 sowie einer Signalverarbeitungseinheit 10 beatmet den Patienten Pt künstlich. Zwischen dem Beatmungsgerät 100 und der patientenseitigen Koppeleinheit 4, 6 ist eine Fluidverbindung hergestellt. Die Beatmungsschläuche zwischen dem Beatmungsgerät 100 und dem Patienten Pt sind nicht dargestellt. Verschiedene Atmungs-Sensoren messen diverse pneumatische oder elektrische oder mechanische Vitalparameter des Patienten Pt und / oder Parameter des Gasflusses zwischen dem Beatmungsgerät 100 und der Lunge Lu des Patienten Pt gucken oder des Gases, welches zur patientenseitigen Koppeleinheit 4, 6 gefördert wird. Um die Offenbarung auszuführen, brauchen nicht notwendigerweise alle diese Atmungs-Sensoren vorhanden zu sein. Folgende Atmungs-Sensoren werden in Figur 1 beispielhaft gezeigt:

- Ein Sensor 15 im oder am Beatmungsgerät 100 misst ein Maß für den Volumenfluss, also für das Volumen Vol' pro Zeiteinheit, des Flusses des Gasgemischs vom Beatmungsgerät 100 zum Patienten Pt (beispielsweise den inspiratorischen Volumenfluss oder das inspiratorische Minutenvolumen) und / oder zurück vom Patienten Pt zum Beatmungsgerät 100 (beispielsweise den exspiratorischen Volumenfluss oder das exspiratorische Minutenvolumen).

- Ein optionaler pneumatischer Sensor 2 umfasst einen Messwertaufnehmer 2.1 mit einer Öffnung, die in der Nähe des Mundes des Patienten Pt angeordnet ist und eine Gasprobe aus der Fluidverbindung abgreift. Die abgegriffene Luft wird über einen nicht gezeigten Schlauch an einen Drucksensor 2.2 übermittelt, und der Drucksensor 2.2 misst ein Maß für den Atemwegsdruck $P_{aw}$ (pressure in airway) in der Fluidverbindung und optional ein Maß für den Volumenfluss Vol'. In einer Ausgestaltung ist der Messwertaufnehmer 2.1 in oder an einem Y-Stück nahe dem Anschlussstück 4 angeordnet, also nahe dem Mund des Patienten Pt. Bevorzugt wird die abgegriffene Gasprobe wieder in die Fluidverbindung eingespeist.

- Eine optionale Sonde 3 in der Speiseröhre Sp des Patienten Pt, bevorzugt umfassend einen Messballon, misst ein Maß für den zeitlich veränderlichen pneumatischen Druck $P_{es}$ (pressure in esophagus) in der Speiseröhre Sp. Die Sonde 3 steht über den Messkatheter 6 in einer Fluidverbindung mit dem Anschlussstück 4 oder ist ein Bestandteil des Messkatheters 6.

- Ein optionaler weiterer Messballon der Sonde 3 oder eine optionale gastrale Sonde 7 in Form eines Messballons, die in den Magen Ma gelegt ist, messen ein Maß für den gastralen Druck $P_{ga}$ im Magen Ma.

- Auf der Brust des Patienten Pt sind mehrere Messelektroden befestigt. Figur 1 zeigt beispielhaft ein herznahes Paar 5.1.1, 5.1.2 von Messelektroden und ein zwerchfellnahes Paar 5.2.1, 5.2.2 von Messelektroden. Mit Hilfe von Messwerten dieser optionalen Messelektroden 5.1.1, ..., 5.2.2 sowie einer nicht gezeigten Referenzelektrode für elektrische Masse werden ein Elektrokardiogramm (EKG) und / oder ein Elektromyogramm (EMG) des Patienten Pt erzeugt, das sind zwei verschiedene respiratorische Signale. Möglich ist, dass auf Basis von Messwerten des herznahen Paars 5.1.1, 5.1.2 ein herznah erzeugtes EKG / EMG und von Messwerten des zwerchfellnahen Paars 5.2.1, 5.2.2 ein zwerchfellnah erzeugtes EKG / EMG generiert werden.

Die Signalverarbeitungseinheit 10 vermag automatisch zu ermitteln, wann Luft oder ein sonstiges Gasgemisch in das Atemsystem des Patienten Pt strömt und wann ein Gasgemisch wieder aus dem Atemsystem strömt, vermag also idealerweise jede Inspirationsphase und jede Exspirationsphase der eigenen Atmungsaktivität des Patienten Pt zu detektieren. Hierfür verwendet die Signalverarbeitungseinheit 10 Messwerte von mindestens einem der Sensoren 2, 3, 7 und 15 und optional von den Messelektroden 5.1.1 bis 5.2.2.

Das Beatmungsgerät 100 führt eine Abfolge von Beatmungshüben durch. Die Signalverarbeitungseinheit 10 löst bei der unterstützenden künstlichen Beatmung wiederholt automatisch die beiden Schritte aus, einen Beatmungshub zu beginnen und ihn wieder zu beenden. Die Signalverarbeitungseinheit 10 steuert einen entsprechenden Aktor (nicht gezeigt) des Beatmungsgeräts 100 an, beispielsweise eine Pumpe oder mindestens ein Ventil, welches mit einem Gebläse zusammenwirkt. Bei jedem Beatmungshub speist das Beatmungsgerät 100 in die Fluidverbindung Atemluft oder ein sonstiges Gasgemisch ein, und dieses Gasgemisch fließt zum Patienten Pt.

Im Ausführungsbeispiel saugt die eigene Atmungsmuskulatur des Patienten Pt ein Gasgemisch an, d.h. der Patient Pt atmet ein Gasgemisch aus der Fluidverbindung ein. Das Einatmen kann durch die spontane Atmung des Patienten Pt hervorgerufen werden. Optional wird seine Atmungsmuskulatur extern stimuliert. Das Beatmungsgerät 100 unterstützt die eigene Atmungsaktivität des Patienten Pt, indem das Beatmungsgerät 100 ein Gasgemisch in die Lunge Lu fördert, wobei dieses Gasgemisch Sauerstoff umfasst.

Im Folgenden wird zwischen den Begriffen "Einatmungs-Vorgang" und "Einatmungs-Anstrengung" des Patienten Pt unterschieden. Die eigene Atmungsmuskulatur des Patienten Pt, die im Körper des Patienten Pt selber angeregt und / oder extern stimuliert wird, versucht, ein Gas anzusaugen. Dieser Versuch wird als Einatmungs-Anstrengung bezeichnet. Gelingt dieser Versuch, fließt also tatsächlich eine relevante Menge des angesaugten Gases in die Lunge Lu, so hat der Patient Pt tatsächlich einen Atemzug und daher auch einen Einatmungs-Vorgang ausgeführt. Möglich ist aber auch, dass eine Einatmungs-Anstrengung nicht einen Einatmungs-Vorgang bewirkt, der zu einem messbaren / relevanten Volumenfluss in den Atemweg des Patienten Pt führt. Diese Situation kann insbesondere dann eintreten, wenn die Lunge Lu des Patienten Pt wenig elastisch ist und daher nach einem Ausatmungs-Vorgang noch relativ viel verbrauchte Luft in der Lunge Lu verbleibt, so dass nur wenig Platz für neues Gas vorhanden ist. Außerdem führt das Beatmungsgerät 100 in manchen Situationen eine Okklusion durch. Bei einer Okklusion wird kurzzeitig verhindert, dass der Patient Pt tatsächlich einatmet. Während einer solchen Okklusion lässt sich in vielen Fällen ein lungenmechanischer Parameter des Patienten Pt besser messen als in eine Situation, in der Gas in die Lunge Lu des Patienten Pt fließt.

Idealerweise löst jede Einatmungs-Anstrengung, den der Patient Pt mit seiner eigenen Atmungsmuskulatur ausführt, unverzüglich einen Beatmungshub des Beatmungsgeräts 100 aus - in einer Ausgestaltung außer bei einer Okklusion. Idealerweise wird dieser Beatmungshub unverzüglich beendet, wenn der Patient Pt die Einatmungs-Anstrengung beendet. Jede Einatmungs-Anstrengung des Patienten Pt löst also idealerweise genau einen Beatmungshub des Beatmungsgeräts 100 aus, und jeder Beatmungshub wird von genau einer Einatmungs-Anstrengung ausgelöst. Die Beatmungshübe des Beatmungsgeräts 100 sind idealerweise also vollständig mit der eigenen Atmungsaktivität des Patienten Pt synchronisiert.

Die eigene Atmungsmuskulatur des Patienten Pt führt Atemzüge aus, bei der zuerst Luft oder ein sonstiges Gasgemisch in die Lunge strömt (Inspiration) und anschließend wieder aus der Lunge strömt (Exspiration). Im Folgenden wird der Begriff "Inspiration" auch für eine Einatmungs-Anstrengung verwendet, die nicht zu einem messbaren Atemzug und somit nicht zu einem messbaren Einatmungs-Vorgang führt.

Die oben beschriebenen Atmungs-Sensoren vermögen jeweils Messwerte zu liefern. Aus Messwerten von mindestens einem Atmungs-Sensor generiert die Signalverarbeitungseinheit 10 ein Signal, welches mit der eigenen Atmungsaktivität des Patienten Pt korreliert. Dieses Signal wird als "respiratorisches Signal" bezeichnet und mit $Sig_{res}$ bezeichnet. In der Regel führen die Messwerte von verschiedenen Atmungs-Sensoren zu unterschiedlichen respiratorischen Signalen. In der Regel weicht die tatsächliche Atmungsaktivität des Patienten Pt wenigstens zeitweise von dem oder jedem gewonnenen respiratorischen Signal $Sig_{res}$ ab, sodass das oder jedes respiratorische Signal $Sig_{res}$ nur eine Näherung für die tatsächliche eigene Atmungsaktivität des Patienten Pt ist.

In einer Ausgestaltung detektiert die Signalverarbeitungseinheit 10 durch Auswertung mindestens eines respiratorischen Signals $Sig_{res}$, dass die eigene Atmungsmuskulatur des Patienten Pt einen Einatmungs-Vorgang oder wenigstens eine Einatmungs-Anstrengung begonnen hat (Beginn einer Inspirationsphase). Die Messwerte, die zu diesem respiratorischen Signal $Sig_{res}$ führen, stammen von dem oder mindestens einem pneumatischen Atmungs-Sensor, beispielsweise von dem pneumatischen Sensor 2 vor dem Mund des Patienten Pt, der Sonde 3 in seiner Speiseröhre Sp oder der gastralen Sonde 7 in seinem Magen. Die Messwerte von diesen pneumatischen Atmungs-Sensoren führen zu jeweils einem pneumatisch gewonnenen respiratorischen Signal $Sig_{res}$.

In einer bevorzugten Ausgestaltung wird mindestens ein pneumatisches respiratorisches Signal $Sig_{res}$ verwendet, welches mit den Einatmungs-Anstrengungen des Patienten Pt korreliert und nicht nur mit den tatsächlich ausgeführten Atemzügen. Die pneumatischen Atmungs-Sensoren 15, 3 und 7 vermögen Messwerte zu liefern, aus denen ein solches respiratorisches Signal $Sig_{res}$ generiert wird. Das respiratorische Signal $Sig_{res}$, welches aus den Messwerten des pneumatischen Atmungs-Sensors 2 generiert wird, korreliert hingegen mit den tatsächlich ausgeführten Atemzügen des Patienten Pt.

In einer anderen Ausgestaltung wird die Tatsache ausgenutzt, dass die eigene Atmungsmuskulatur des Patienten Pt durch eine Abfolge von elektrischen Impulsen, die im Körper des Patienten Pt und / oder optional von einem stimulierenden Gerät erzeugt werden, zu Bewegungen angeregt wird. Diese elektrischen Impulse lassen sich messen und führen zu einem elektrischen Signal. Dieses mit der internen und / oder externen Stimulation korrelierende elektrische Signal für die Atmungsmuskulatur wird als ein "elektrisches respiratorisches Signal" bezeichnet und lässt sich näherungsweise messen. Die Herzaktivität des Patienten Pt wird durch eine weitere Abfolge von elektrischen Signalen, die im Körper des Patienten Pt erzeugt werden, ausgelöst. Aus dieser Abfolge lässt sich ein Signal für die Herzaktivität generieren, welches als ein "kardiogenes Signal" bezeichnet wird.

Für die Messwerte der Messelektroden 5.1.1 bis 5.2.2 sowie die Messwerte der nicht gezeigten Referenzelektrode wird in einer Ausführungsform eine Signal-Vorverarbeitung durchgeführt, welche bevorzugt eine Summenbildung und eine Glättung der gemessenen elektrischen Messwerte umfasst und eine sogenannte Hüllkurve (envelope) liefert. Diese Hüllkurve ist oder liefert ein Summen-Signal $Sig_{Sum}$, welches aus einer Überlagerung des elektrischen respiratorischen Signal mit dem kardiogenen Signal resultiert und von Störsignalen beeinflusst sein kann. Störsignale können aus dem Körper des Patienten Pt und aus der Umgebung kommen. Der Einfluss des kardiogenen Signals auf das Summen-Signal $Sig_{Sum}$ wird wenigstens näherungsweise rechnerisch kompensiert. Beispielsweise werden im Summen-Signal $Sig_{Sum}$ diejenigen Abschnitte erkannt, die von jeweils einem Herzschlag stammen, beispielsweise jeweils ein sogenannter QRS-Abschnitt. Oder vom Summen-Signal wird ein standardisierter Verlauf des kardiogenen Signals im Verlaufe eines einzigen Herzschlags subtrahiert, ein sogenanntes EKG-Template. Die rechnerische Kompensation liefert eine Näherung für das elektrische respiratorische Signal $Sig_{res}$. Die auf diese Weise gewonnene Schätzung für das elektrische respiratorische Signal $Sig_{res}$ zeigt ebenfalls an, wann der Patient Pt eine Einatmungs-Anstrengung beginnt und wann der Patient Pt sie beendet.

Möglich ist, dass aus Messwerten des herznahen Paars 5.1.1, 5.1.2 ein erstes elektrisches respiratorisches Signal $Sig_{res}$ gewonnen wird und aus Messwerten des zwerchfellnahen Paars 5.2.1, 5.2.2 ein zweites elektrisches respiratorisches Signal. In der Regel unterscheiden sich diese beiden elektrischen Signale voneinander.

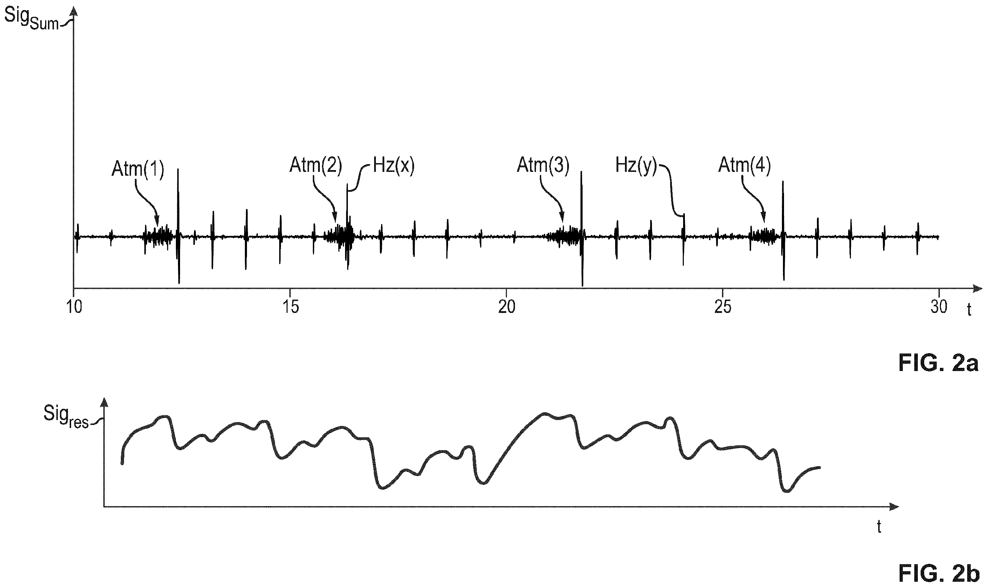

Figur 2 a) zeigt beispielhaft ein elektrisches Summen-Signal $Sig_{Sum}$, das aus Messwerten der beiden Paare 5.1.1, 5.1.2 und 5.2.1, 5.2.2 von Messelektroden sowie Messwerten der nicht gezeugten Referenzelektrode gewonnen wurde. Auf der x-Achse ist die Zeit t aufgetragen, auf der y-Achse der jeweilige Wert des Summen-Signals $Sig_{Sum}$. Zu sehen ist die jeweilige Auswirkung einer Abfolge von Herzschlägen, z.B. die Herzschläge Hz(x) und Hz(y), sowie einer Abfolge von Atemzügen, z.B. die Atemzüge Atm(1), ..., Atm(4). Aus diesem elektrischen Summen-Signal $Sig_{Sum}$ lässt sich ein elektrisches respiratorisches Signal $Sig_{res}$ generieren, bevorzugt indem der Einfluss des kardiogenen Signals auf das Summen-Signal $Sig_{Sum}$ rechnerisch kompensiert wird. Figur 2 b) zeigt ein beispielhaftes pneumatisches respiratorisches Signal $Sig_{res}$, welches durch Auswertung von Messwerten einer der Sensoren 3, 7, 15 generiert wurde

In einer nicht gezeigten weiteren Ausgestaltung liefern Messwerte eines mechano-myographischen Sensors ein mechanisches respiratorisches Signal, welches mit der Aktivität der eigenen Atmungsmuskulatur des Patienten Pt korreliert.

Die Signalverarbeitungseinheit 10 detektiert in mindestens einem respiratorischen Signal $Sig_{res}$ den jeweiligen Beginn und das jeweilige Ende jeder Einatmungs-Anstrengung. Die gerade beschriebenen Ausgestaltungen, um auf unterschiedliche Weisen respiratorische Signale zu generieren, lassen sich miteinander kombinieren. In einer Ausgestaltung liegen mindestens zwei verschiedene respiratorische Signale vor. Diese respiratorischen Signale stimmen idealerweise überein, differieren in der Praxis aber in der Regel voneinander und von den tatsächlichen Einatmungs-Anstrengungen. In einer Realisierungsform erzeugt die Signalverarbeitungseinheit 10 aus mehreren respiratorischen Signalen ein gemitteltes respiratorisches Signal $Sig_{res}$ und verwendet dieses gemittelte respiratorische Signal $Sig_{res}$, um die Einatmungs-Anstrengungen des Patienten Pt zu detektieren. In einer anderen Realisierungsform verwendet die Signalverarbeitungseinheit 10 hingegen mehrere respiratorische Signale, um die Einatmungs-Anstrengungen des Patienten Pt zu detektieren.

Idealerweise ist jede Einatmungs-Anstrengung oder wenigstens jeder Einatmungs-Vorgang des Patienten Pt in dem oder jedem respiratorischen Signal $Sig_{res}$ sichtbar, außerdem jeder Ausatmungs-Vorgang. Idealerweise detektiert die Signalverarbeitungseinheit 10 jede Einatmungs-Anstrengung in jedem respiratorischen Signal $Sig_{res}$ und detektiert stets diejenigen Zeitpunkte, an denen die Einatmungs-Anstrengung beginnt bzw. endet. Idealerweise sind die Beatmungshübe des Beatmungsgeräts 100 ideal mit den Einatmungs-Anstrengungen des Patienten Pt synchronisiert.

In der Praxis kann es vorkommen, dass eine Einatmungs-Anstrengung in dem oder mindestens einem respiratorischen Signal $Sig_{res}$ überhaupt nicht detektiert wird. Weiterhin ist möglich, dass die Signalverarbeitungseinheit 10 zwar dieselbe Einatmungs-Anstrengung des Patienten Pt in mehreren respiratorischen Signalen $Sig_{res}$ detektiert, aber mit unterschiedlichen Zeitpunkten für den Beginn und / oder für das Ende des Einatmens. Die unterschiedlichen Schätzungen können zu einem falschen Zeitpunkt zusammengefasst werden oder sogar fälschlich als zwei verschiedene Einatmungs-Anstrengungen eingestuft werden. Möglich ist auch, dass die Signalverarbeitungseinheit 10 in dem oder einem respiratorischen Signal $Sig_{res}$ vermeintlich eine Einatmungs-Anstrengung detektiert, obwohl der Patient Pt zu diesem Zeitpunkt keine Einatmungs-Anstrengung ausgeführt hat. Das oder jedes respiratorische Signal $Sig_{res}$ weicht nämlich zwangsläufig von der tatsächlichen eigenen Atmungsaktivität des Patienten Pt ab. Einige Gründe hierfür sind: Zwischen dem Zwerchfell Zw und weiteren Regionen des Körpers des Patienten Pt, welche die Einatmungs-Anstrengungen bewirken, und dem Messpunkt, an dem die Messwerte für ein respiratorisches Signal $Sig_{res}$ gemessen werden, tritt ein Abstand auf, wodurch Störsignale wirksam werden können. Außerdem treten in der Regel Messungenauigkeiten und Messfehler der verwendeten Sensoren auf, und weitere Signale, die im Körper des Patienten Pt oder auch außerhalb erzeugt werden, können auf das gewonnene respiratorische Signal $Sig_{res}$ einwirken. Möglich ist auch, dass die Signalverarbeitungseinheit 10 eine Einatmungs-Anstrengung des Patienten Pt überhaupt nicht erkennt.

Bevorzugt detektiert die Signalverarbeitungseinheit 10 dann eine Einatmungs-Anstrengung des Patienten Pt, wenn diese Einatmungs-Anstrengung in dem oder mindestens einem respiratorischen Signal $Sig_{res}$ sichtbar wird, und zwar auch dann, wenn diese Einatmungs-Anstrengung in einem anderen respiratorischen Signal $Sig_{res}$ nicht sichtbar wird. Falls dieselbe Einatmungs-Anstrengung im mehreren respiratorischen Signalen sichtbar wird, so ermittelt die Signalverarbeitungseinheit 10 bevorzugt in mindestens zwei dieser respiratorischen Signale den jeweiligen Zeitpunkt für den Beginn und das Ende des Einatmens. Falls mehrere respiratorische Signale vorliegen, so mittelt in einer Realisierungsform die Signalverarbeitungseinheit 10 über die detektierten Zeitpunkte für den Beginn der Einatmungs-Anstrengung und mittelt über die detektierten Zeitpunkte für das Ende der Einatmungs-Anstrengung und detektiert dadurch jeweils einen gemittelten Beginn-Zeitpunkt und einen gemittelten Ende-Zeitpunkt für jede Einatmungs-Anstrengung. Bei dieser Mittelung werden in einer Ausgestaltung Gewichtsfaktoren berücksichtigt, wobei ein Gewichtsfaktor umso größer ist, je zuverlässiger ein bestimmter Atmungs-Sensor und / oder je zuverlässiger die Herleitung des respiratorischen Signals von diesem Sensor ist. Möglich ist auch, dass die Zeitpunkte in demjenigen respiratorischen Signal verwendet werden, das aktuell die höchste Zuverlässigkeit aufweist.

In der Regel verstreicht zwischen dem Zeitpunkt, an dem der Patient Pt eine Einatmungs-Anstrengung beginnt oder beendet, und dem Zeitpunkt, an dem dieses Ereignis in einem pneumatischen respiratorischen Signal $Sig_{res}$ detektiert wird, eine Zeitspanne, insbesondere wenn der verwendete pneumatische Atmungs-Sensor sich außerhalb des Körpers des Patienten Pt befindet. Ein Grund ist, dass die Einatmungs-Anstrengung zu einem hinreichend großen Volumenfluss an der jeweiligen Messstelle eines pneumatischen Atmungs-Sensors geführt haben muss, bevor die Einatmungs-Anstrengung im respiratorischen Signal $Sig_{res}$ von diesem Atmungs-Sensor detektiert wird. Zwischen dem Zeitpunkt, an dem der Körper des Patienten Pt oder auch ein stimulierendes Gerät elektrische Impulse generiert, welche die eigene Atmungsmuskulatur aktivieren und welche gemessen werden, und dem Zeitpunkt, zu dem die eigene Atmungsmuskulatur tatsächlich eine Einatmungs-Anstrengung beginnt, verstreicht außerdem ebenfalls Zeit. Diese Zeitspanne kann von der sogenannten neuromuskulären Effizienz der eigenen Atmungsmuskulatur abhängen, also wie rasch und wie gut die eigene Atmungsmuskulatur des Patienten Pt auf stimulierende elektrische Impulse reagiert. Diese beiden Zeitspannen lassen sich in vielen Fällen schätzen, aber jede Schätzung kann mit Unsicherheit behaftet sein.

Die tatsächliche eigene Atmungsaktivität und daher auch das oder jedes respiratorische Signal $Sig_{res}$ oszillieren in der Regel, wobei in der Regel die Amplitude und die Frequenz dieser Oszillation über der Zeit variiert. Die Signalverarbeitungseinheit 10 des Beatmungsgeräts 100 wendet auf das oder jedes gewonnene respiratorische Signal $Sig_{res}$ automatisch eine Entscheidungsvorschrift an, um im respiratorischen Signal $Sig_{res}$ den Beginn und das Ende einer Einatmungs-Anstrengung des Patienten Pt zu detektieren und abhängig vom detektierten Beginn und vom detektierten Ende der detektierten Einatmungs-Anstrengung einen Beatmungshub zu beginnen bzw. zu beenden. Diese Entscheidungsvorschrift wendet in vielen Fällen Parameter an, beispielsweise die folgenden Betriebsparameter:

- Wenn ein Maß für den Volumenfluss von Atemluft zur Lunge Lu des Patienten Pt größer als eine vorgegebene Schranke x ist, optional innerhalb einer vorgegebenen Zeitspanne T größer als die Schranke x ist, so wird entschieden, dass der Patient Pt eine Einatmungs-Anstrengung begonnen hat, und ein neuer Beatmungshub wird begonnen.
- Wenn das Maß für diesen Volumenfluss kleiner wird als ein Prozentsatz y des zuletzt gemessenen maximalen Volumenflusses, also des maximalen Volumenflusses bei dieser Einatmungs-Anstrengung, so wird entschieden, dass der Patient Pt die Einatmungs-Anstrengung beendet hat, und der aktuelle Beatmungshub wird beendet.

Werte für diese Betriebsparameter x, y werden aufgrund folgender einander widersprechenden Anforderungen festgelegt:

- Jede Einatmungs-Anstrengung soll detektiert werden.
- Der Beginn und das Ende jeder Einatmungs-Anstrengung, den der Patient Pt tatsächlich ausführt, sollen möglichst genau detektiert werden.
- Bei der unterstützenden Beatmung soll nur dann ein Beatmungshub ausgeführt werden, wenn auch der Patient Pt eine Einatmungs-Anstrengung ausführt. Vermieden werden soll, dass fälschlicherweise ein Beatmungshub aufgrund einer vermeintlichen Einatmungs-Anstrengung ausgeführt wird, obwohl der Patient Pt gerade nicht einatmet. Insbesondere soll in der Regel vermieden werden, dass das Beatmungsgerät 100 einen Beatmungshub ausführt, während der Patient Pt ausatmet.

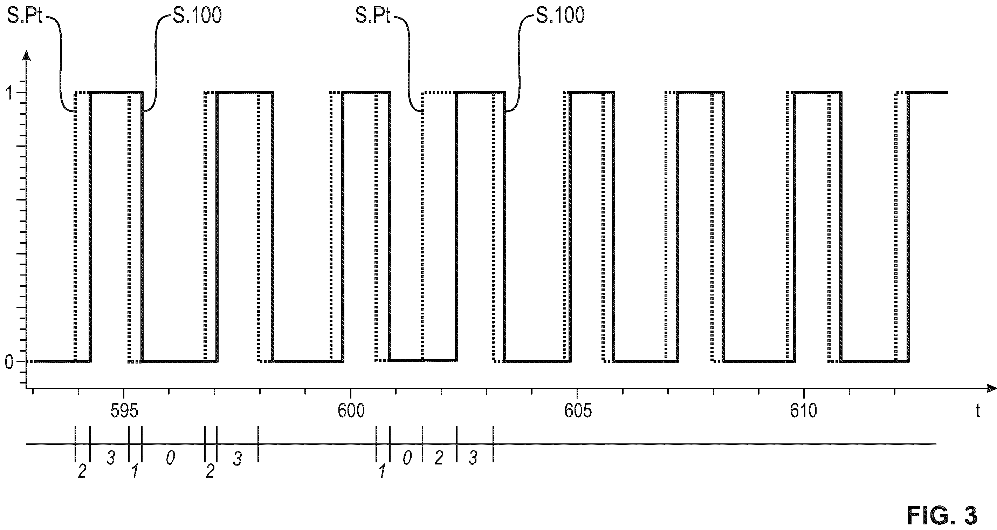

Figur 3 veranschaulicht mittels eines beispielhaften schematischen Diagramms eine Situation, bei der die Beatmungshübe des Beatmungsgeräts 100 der eigenen Atmungsaktivität des Patienten Pt "hinterherhinken". Auf der x-Achse ist die Zeit in [sec] aufgetragen. Zeitspannen, in denen die eigene Atmungsmuskulatur des Patienten Pt eine Einatmungs-Anstrengung bewirkt, also versucht, Luft anzusaugen, sind im Verlauf S.Pt (gestrichelt dargestellt) mit einer 1 codiert, die übrigen Zeitspannen mit einer 0. Entsprechend sind Zeitspannen, in denen das Beatmungsgerät 100 Atemluft zum Patienten Pt fördert, also einen Beatmungshub ausführt, im Verlauf S.100 (durchgezogen dargestellt) mit einer 1 codiert, die übrigen Zeitspannen mit einer 0.

In dem gezeigten Beispiel werden die Beatmungshübe abhängig von einem pneumatischen respiratorischen Signal $Sig_{res}$ begonnen und beendet, also abhängig von Messwerten mindestens eines pneumatischen Sensors 15, 3, 7. Im gezeigten Beispiel beginnt und endet jeder Beatmungshub später als die Einatmungs-Anstrengung, die diesen Beatmungshub auslöst. Verschiedene mögliche Gründe dafür, dass die unterstützende künstliche Beatmung hinter der eigenen Atmungsaktivität "hinterherhinkt", wurden bereits oben genannt.

Möglich ist auch, dass ein Beatmungshub früher beginnt als die Einatmungs-Anstrengung, der diesen Beatmungshub auslöst. Diese Situation kann insbesondere dann auftreten, wenn der Beatmungshub durch eine Schätzung für ein elektrisches respiratorisches Signal $Sig_{res}$ ausgelöst wird, wobei dieses elektrische Signal $Sig_{res}$ abhängig von elektrischen Impulsen, die im Körper des Patienten Pt erzeugt werden, generiert wird und nicht durch den Beginn einer ausgeführten Einatmungs-Anstrengung. Eine mögliche Ursache dafür, dass ein Beatmungshub zu früh beginnt, ist die folgende: Ein Schwellwert, um ein elektrisches respiratorisches Signal $Sig_{res}$ für eine Einatmungs-Anstrengung des Patienten Pt zu erkennen, ist zu niedrig eingestellt, d.h. der Sensor ist zu empfindlich. Mögliche Gründe dafür, dass die unterstützende künstliche Beatmung der eigenen Atmungsaktivität vorauseilt, wurden ebenfalls oben genannt.

Zu jedem Abtast-Zeitpunkt können die folgenden vier Situationen auftreten, die in einer bevorzugten Ausgestaltung mit vier unterschiedlichen Werten codiert werden. Die nachfolgende Tabelle 1 zeigt diese vier Situationen und beispielhaft deren jeweilige Codierung.

13

*Tabelle 1*

| Codierung | Beatmungshub ausgeführt (Luft fließt vom Beatmungsgerät 100 zum Patienten Pt)? | Einatmungs-Anstrengung ausgeführt (eigene Atmungsmuskulatur versucht, Atemluft anzusaugen)? |
|---|---|---|
| 0 | Nein | Nein |
| 1 | Ja | Nein |
| 2 | Nein | Ja |
| 3 | Ja | Ja |

Selbstverständlich sind auch andere Codierungen möglich als die Ziffern 0,1, 2, 3.

Im Folgenden werden abkürzend die Bezeichnungen "Situation 0" bis "Situation 3" verwendet. Diese Codierungen sind beispielhaft unter der x-Achse in Figur 3 eingetragen.

Nachfolgend wird eine Abfolge von drei Situationen als "Sequenz" bezeichnet, vorausgesetzt jeweils zwei unmittelbar aufeinander folgende Situationen unterscheiden sich voneinander.

Falls das Beatmungsgerät 100 ideal mit der eigenen Atmungsaktivität des Patienten Pt synchronisiert ist, treten nur die Situationen 0 und 3 auf. Ein ideal synchronisierter Beatmungshub führt zu der Situationen-Sequenz [0,3,0]. In der Praxis treten in der Regel auch die Situationen 1 und 2 auf. Diese Situationen bedeuten eine Asynchronie.

In der Regel ist es für den Patienten Pt völlig unschädlich, wenn der Beginn und das Ende eines Beatmungshubs um nicht mehr als eine vorgegebene Zeitdauer-Schranke von dem Beginn bzw. dem Ende der Einatmungs-Anstrengung, die diesen Beatmungshub auslöst, abweicht. Diese Zeitdauer-Schranke kann für alle Patienten Pt und alle Situationen fest vorgegeben sein und beispielsweise 100 msec betragen. Die Zeitdauer-Schranke kann auch von gemessenen Vitalparametern des Patienten Pt und / oder davon, auf welche Weise die Beatmungshübe von den Einatmungs-Anstrengungen differieren, abhängen. Daher wird im Folgenden nur dann eine Situation 1 oder 2 registriert, wenn diese Situation 1 oder 2 länger als die vorgegebene Zeitdauer-Schranke andauert. Dies lässt sich beispielsweise mit einer ausreichend großen Abtast-Frequenz oder mit einer Signal-Vorverarbeitung sicherstellen.

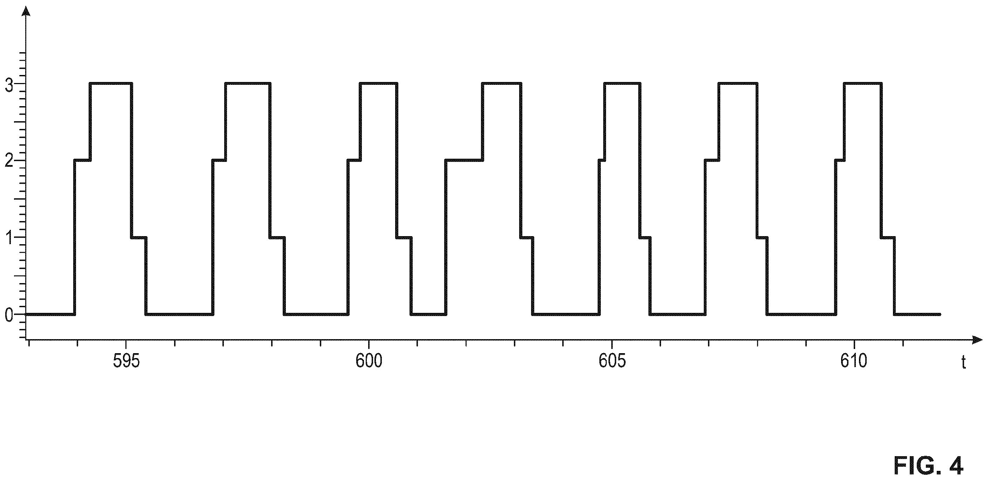

Die Situationen 1 und 2 werden als "Asynchronie-Situationen" bezeichnet. Figur 4 zeigt den zeitlichen Verlauf der vier Situationen 0, 1, 2, 3 für den Verlauf von Figur 3. Außerdem sind in Figur 3 beispielhaft einige Zeitspannen eingetragen, in denen jeweils eine dieser vier Situationen auftritt. Auf der x-Achse von Figur 4 ist wiederum die Zeit t in [sec] aufgetragen, auf der y-Achse der Wert 0, 1, 2, 3 für die jeweilige Situation.

Eine Abfolge von Abtast-Zeitpunkten ist vorgegeben. Für jeden Abtast-Zeitpunkt wird automatisch festgestellt, welche der vier möglichen Situationen 0, 1, 2, 3 an diesem Abtast-Zeitpunkt vorliegt. Dieses Vorgehen liefert eine Situationen-Abfolge. Wie bereits dargelegt, wird ein Abschnitt der Situationen-Abfolge, der aus drei unmittelbar aufeinanderfolgenden Situationen besteht, als eine "Sequenz" bezeichnet, vorausgesetzt zwei unmittelbar aufeinanderfolgende Situationen unterscheiden sich voneinander. Beispiele für Situationen-Sequenzen sind [0,1,0], [2,3,0] und [1,0,3]. Bei n = 4 verschiedenen möglichen Situationen gibt es

$$n * (n-1) * (n-1) = 36$$

verschiedene mögliche Situationen-Sequenzen.

Als eine "Asynchronie-Sequenz" wird eine Situationen-Sequenz bezeichnet, bei der die zeitlich mittlere Situation eine Asynchronie-Situation ist, also gleich 1 oder gleich 2 ist. Die Offenbarung ermöglicht es, die Asynchronie-Sequenzen während einer unterstützenden künstlichen Beatmung des Patienten Pt gezielt zu detektieren. Bei n = 4 verschiedenen möglichen Situationen und m = 2 Asynchronie-Situationen gibt es

$$(n - 1) * m * (n-1) = 18$$

verschiedene mögliche Asynchronie-Sequenzen.

Die folgende Tabelle 2 zeigt beispielhaft, welche dieser 18 möglichen Asynchronie-Sequenzen welche technischen Bedeutungen haben.

*Tabelle 2*

| Sequenz | technische Bedeutung |
|---|---|
| [0,1,0] | Beatmungshub ohne eine zeitlich überlappende Einatmungs-Anstrengung ausgeführt |

(fortgesetzt)

| Sequenz | technische Bedeutung |
|---------|----------------------|
| [0,1,2] | zuerst weder Beatmungshub noch Einatmungs-Anstrengung, dann nur Beatmungshub ausgeführt, dann nur Einatmungs-Anstrengung ausgeführt |
| [0,1,3] | Beatmungshub vor Einatmungs-Anstrengung begonnen |
| [0,2,0] | Einatmungs-Anstrengung ohne einen zeitlich überlappenden Beatmungshub ausgeführt |
| [0,2,1] | zuerst nur Einatmungs-Anstrengung, unmittelbar danach nur Beatmungshub ausgeführt |
| [0,2,3] | Einatmungs-Anstrengung vor Beatmungshub begonnen |
| [1,2,0] | Beatmungshub vor Einatmungs-Anstrengung beendet |
| [1,2,1] | Einatmungs-Anstrengung während eines Beatmungshubs begonnen und beendet |
| [1,2,3] | Beatmungshub vor Einatmungs-Anstrengung begonnen |
| [2,1,0] | Einatmungs-Anstrengung vor Beatmungshub beendet |
| [2,1,2] | Beatmungshub während Einatmungs-Anstrengung begonnen und beendet |
| [2,1,3] | Einatmungs-Anstrengung vor Beatmungshub begonnen |
| [3,1,0] | Einatmungs-Anstrengung vor Beatmungshub beendet |
| [3,1,2] | erste Einatmungs-Anstrengung vor Beatmungshub beendet, dann zweite Einatmungs-Anstrengung ohne Beatmungshub begonnen |
| [3,1,3] | erste Einatmungs-Anstrengung während eines Beatmungshubs beendet und zweite Einatmungs-Anstrengung während desselben Beatmungshubs begonnen |
| [3,2,0] | Beatmungshub vor Einatmungs-Anstrengung beendet |
| [3,2,1] | erster Beatmungshub vor Einatmungs-Anstrengung beendet, zweiter Beatmungshub ohne Einatmungs-Anstrengung begonnen |
| [3,2,3] | erster Beatmungshub während einer Einatmungs-Anstrengung beendet und zweiter Beatmungshub während derselben Einatmungs-Anstrengung begonnen |

Die folgende Tabelle 3 zeigt die klinischen Bedeutungen für acht relativ häufige Arten von Asynchronie-Sequenzen. Diese acht Arten von Asynchronie-Sequenzen fungieren als acht mögliche Asynchronie-Arten im Sinne der Patentansprüche.

*Tabelle 3*

| Sequenz | klinische Bedeutung (deutsch) | klinische Bedeutung (englisch) |
|---------|-------------------------------|--------------------------------|
| [0,1,0] | Beatmungshub von allein ausgelöst | Auto Triggering |
| [0,1,3] | Beatmungshub löst Einatmungs-Anstrengung aus | Reverse Triggering |
| [0,2,0] | Einatmungs-Anstrengung verpasst | Missed Effort |
| [0,2,3] | Beatmungshub zu spät ausgelöst | Late Triggering |
| [3,1,0] | Beatmungshub zu spät beendet | Late Cycling Off |
| [3,1,3] | Ende einer Einatmungs-Anstrengung verpasst (Ausatmung verpasst) | Missed Expiration |
| [3,2,0] | Beatmungshub zu früh beendet | Premature Cycling Off |
| [3,2,3] | neuer Beatmungshub während Einatmungs-Anstrengung ausgelöst | Double Triggering |

Zwei Klassen von Asynchronie-Sequenzen lassen sich unterscheiden, nämlich Zeit-Asynchronien und Ereignis-Asynchronien (Dyssynchronien). Bei einer Zeit-Asynchronie beginnt der Beatmungshub später oder früher als die Einatmungs-Anstrengung, die diesen Beatmungshub auslöst. Falls bei der unterstützenden künstlichen Beatmung nur Zeit-Asynchronien auftreten, löst jede Einatmungs-Anstrengung des Patienten Pt jeweils genau einen Beatmungshub des Beatmungsgeräts 100 aus. Bei einer Ereignis-Asynchronie löst eine Einatmungs-Anstrengung gar keinen Beatmungshub aus, oder ein Beatmungshub wird ohne eine Einatmungs-Anstrengung ausgelöst. Oder eine Einatmungs-Anstrengung des Patienten Pt wird zwar durch einen Beatmungshub unterstützt, aber der Patient unterbricht die Einatmungs-Anstrengung, oder das Beatmungsgerät 100 unterbricht den unterstützenden Beatmungshub. Die vier relativ häufigen Asynchronie-Sequenzen [0,1,3], [0,2,3], [3,2,0] und [3,1,0] sind Zeit-Asynchronien, die übrigen vier relativ häufigen Asynchronie-Sequenzen [0,2,0], [3,1,3], [0,1,0] und [3,2,3] Ereignis-Asynchronien.

Eine signalverarbeitende Überwachungseinheit 11, welche die unterstützende künstliche Beatmung durch das Beatmungsgerät 100 überwacht und einen Prozessor und einen Datenspeicher umfasst, empfängt das generierte respiratorische Signal $Sig_{res}$, optional jedes einzelne respiratorische Signal $Sig_{res}$ von verschiedenen Atmungs-Sensoren, und detektiert in dem oder jedem gewonnenen respiratorischen Signal $Sig_{res}$ die Einatmungs-Anstrengungen des Patienten Pt und für jede Einatmungs-Anstrengung dessen Beginn und dessen Ende. Weil diese Überwachungseinheit 11 das Beatmungsgerät 100 überwacht, aber selber keine Beatmungshübe auslöst, kann die Überwachungseinheit 11 einen längeren Abschnitt des oder jedes respiratorischen Signals $Sig_{res}$ auswerten. Für diese Auswertung steht der Überwachungseinheit 11 eine größere Rechenzeit zur Verfügung, als sie der Signalverarbeitungseinheit 10 zur Verfügung steht, um Beatmungshübe auszulösen. Die Überwachungseinheit 11 kann ein Bestandteil des Beatmungsgeräts 100 sein oder auch außerhalb des Beatmungsgeräts 100 angeordnet sein. Möglich ist aber auch, dass dasselbe signalverarbeitende Gerät sowohl die Funktionen der Signalverarbeitungseinheit 10 als auch die Funktionen der Überwachungseinheit 11 ausführt.

Die Überwachungseinheit 11 wendet in einer Ausgestaltung ein lernendes Verfahren auf den jeweils zeitlich letzten Abschnitt des respiratorischen Signals $Sig_{res}$ an, um den Beginn und das Ende einer Einatmungs-Anstrengung zu detektieren. In einer Realisierungsform wird das lernende Verfahren auf die N zeitlich letzten detektierten Einatmungs-Anstrengungen angewendet, wobei N eine vorgegebene Anzahl ist. In einer anderen Realisierungsform wird das lernende Verfahren auf den zeitlich jüngsten Abschnitt des respiratorischen Signals $Sig_{res}$ angewendet, wobei dieser jüngste Abschnitt eine vorgegebene Zeitdauer T aufweist. In vielen Fällen vergrößert die Anwendung eines lernenden Verfahrens die Zuverlässigkeit, mit der im respiratorischen Signal $Sig_{res}$ der jeweilige Beginn und das jeweilige Ende der Einatmungs-Anstrengungen detektiert wird.

Außerdem empfängt die Überwachungseinheit 11 von der Signalverarbeitungseinheit 10 für jeden Beatmungshub eine Kennzeichnung derjenigen beiden Zeitpunkte, an denen dieser Beatmungshub begonnen bzw. beendet wurde.

Die Überwachungseinheit 11 vergleicht das empfangene oder durch Mittelung oder durch eine andere geeignete Art der Signalverarbeitung erzeugte respiratorische Signal $Sig_{res}$, welches mit der eigenen Atmungsaktivität des Patienten Pt korreliert, mit dem zeitlichen Verlauf der Beatmungshübe, die das Beatmungsgerät 100 ausführt und die beispielhaft durch die Kurve S.100 in Figur 3 gezeigt werden. Die Überwachungseinheit 11 detektiert durch den Vergleich der beiden Kurven jede mögliche Asynchronie-Sequenz, die beispielhaft in Tabelle 3 gezeigt wird. Bevorzugt wird hierbei eine Asynchronie-Situation 1 oder 2 nur dann berücksichtigt, wenn diese länger als die vorgegebene Zeitdauer-Schranke von beispielsweise 100 msec oder von variabler Dauer andauert. Die Überwachungseinheit 11 erzeugt eine zeitliche Abfolge n1 n2 n3 ..., wobei n1, n2, n3 Codierungen für die möglichen Situationen sind, hier also jeweils eine Zahl 0, 1, 2 oder 3. Diese Abfolge wird im Folgenden als "Situationen-Abfolge" bezeichnet.

Bevorzugt zählt die Überwachungseinheit 11 automatisch, wie oft in der Situationen-Abfolge welche Asynchronie-Sequenz auftritt. In manchen Fällen ist eine bestimmte Asynchronie-Sequenz nur dann relevant, wenn ihre Häufigkeit in der Situationen-Abfolge oberhalb einer vorgegebenen Häufigkeits-Schranke liegt oder in der Situationen-Abfolge einem bestimmten Auftretens-Muster folgt. Dies gilt insbesondere für die Asynchronie-Sequenz [0,1,3] (Reverse Triggering), d.h. ein Beatmungshub löst eine Einatmungs-Anstrengung aus, anstelle dass eine Einatmungs-Anstrengung einen Beatmungshub auslöst.

Wie bereits dargelegt wurde, wendet die Signalverarbeitungseinheit 10 eine Entscheidungsvorschrift an, um in dem respiratorischen Signal $Sig_{res}$ den jeweiligen Beginn und das jeweilige Ende jeder Einatmungs-Anstrengung zu detektieren. Diese Entscheidungsvorschrift hängt von mindestens einem Parameter ab. In einer Ausgestaltung weist die Überwachungseinheit 11 automatisch diesem Parameter einen Wert zu oder verändert einen bereits zugewiesenen Wert, und zwar abhängig davon, wie oft und / oder wie lange eine mögliche Asynchronie-Sequenz tatsächlich aufgetreten ist. Falls beispielsweise einzelne Einatmungs-Anstrengungen nicht detektiert werden, so wird bevorzugt eine untere Schranke für den Volumenfluss in der Entscheidungsvorschrift abgesenkt. Falls umgekehrt einzelne Beatmungshübe ohne eine entsprechende Einatmungs-Anstrengung ausgelöst werden, so wird diese untere Volumenfluss-Schranke angehoben.

Die Überwachungseinheit 11 veranlasst im Ausführungsbeispiel, dass das Ergebnis der Überwachung auf der Anzeige- und Bedieneinheit 12 angezeigt wird. In vielen Fällen ist mindestens eine der Randbedingungen zu beachten, die häufig im klinischen Alltag auftreten, nämlich dass

- die Anzeige- und Bedieneinheit 12 relativ klein ist,
- trotzdem relativ viele Informationen dargestellt werden müssen oder sollen,
- manchmal keine optimale Beleuchtung vorliegt und
- ein Benutzer die dargestellten Ergebnisse rasch erfassen können soll, auch unter hoher Belastung und / oder unter Reizüberflutung, die beide oft im klinischen Alltag vorkommen.

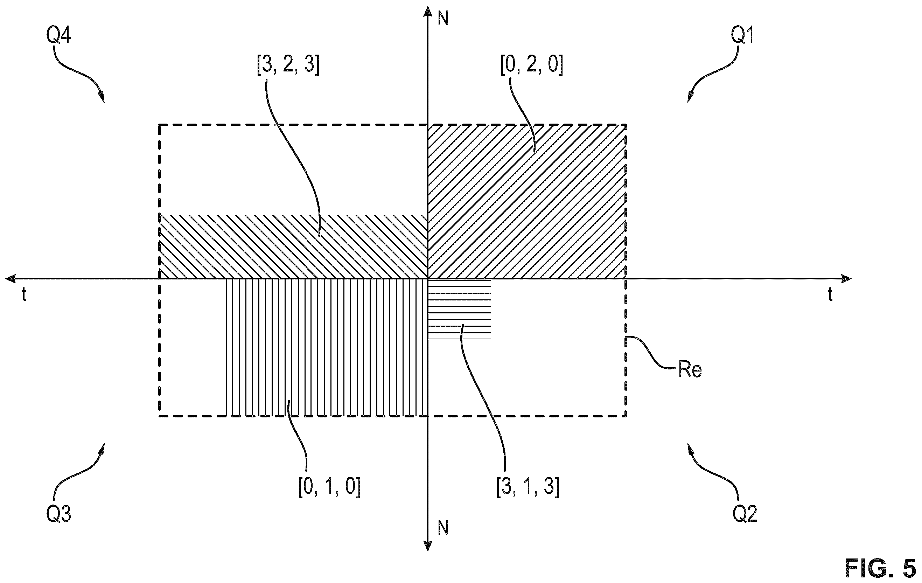

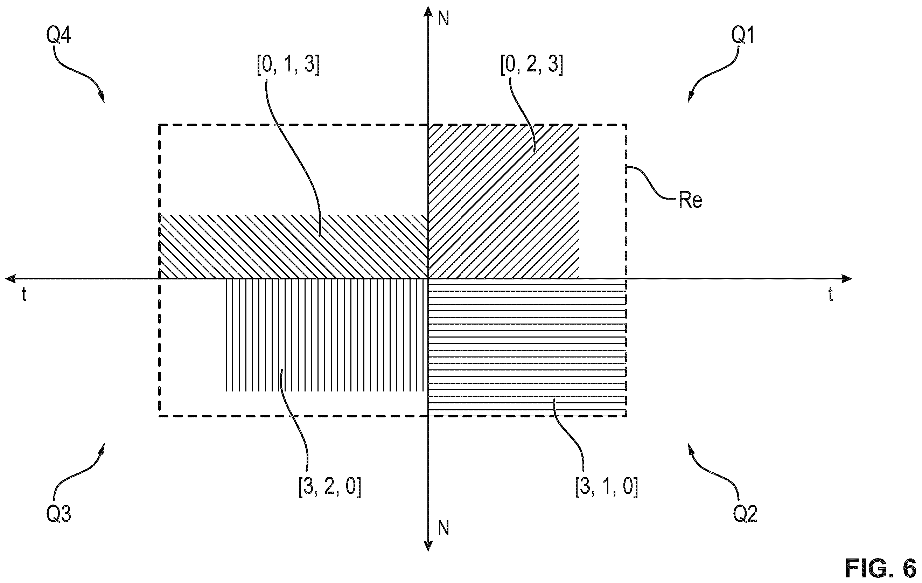

Figur 5 und Figur 6 zeigen beispielhaft, wie ein Ergebnis, welches die Überwachungseinheit 11 erzielt hat, auf der Anzeige- und Bedieneinheit 12 angezeigt wird. Die obere Hälfte der Darstellung zeigt inspiratorische Asynchronie-Sequenzen, die untere Hälfte exspiratorische Asynchronie-Sequenzen. Bei einer inspiratorischen Asynchronie-Sequenz wird eine Einatmungs-Anstrengung nicht vollständig von einem Beatmungshub zeitlich überlagert, bei einer exspiratorischen Asynchronie-Sequenz wird ein Beatmungshub nicht vollständig von einer Einatmungs-Anstrengung zeitlich überlagert. Die linke Hälfte der Darstellung bezieht sich auf Asynchronien, die durch eine zu früh oder fälschlich ausgelöste Aktivität des Beatmungsgeräts 100 verursacht werden. Die rechte Hälfte bezieht sich entsprechend auf eine zu spät oder fehlende Aktivität des Beatmungsgeräts 100 verursacht werden. Die Bezeichnungen "zu früh", "zu spät", "fälschlicherweise" und "fehlend" beziehen sich auf die Sicht des Patienten Pt.

Figur 5 zeigt beispielhaft, wie die vier Ereignis-Asynchronien dargestellt werden, Figur 6 beispielhaft, wie die vier Zeit-Asynchronien dargestellt werden. In dem gezeigten Beispiel ist die genutzte Anzeigefläche in vier Quadranten Q1 bis Q4 unterteilt. Sowohl in Figur 5 als auch in Figur 6 stehen die Quadranten Q1 und Q4 für zwei verschiedene inspiratorische Asynchronie-Sequenzen, die beiden anderen Quadranten Q2 und Q3 für zwei verschiedene exspiratorische Asynchronie-Sequenzen. Selbstverständlich lassen sich die Asynchronie-Sequenzen auch anders auf die vier Quadranten verteilen.

Die Quadranten Q3 und Q4 in Figur 6 stehen für Asynchronie-Sequenzen aufgrund von zu früh ausgeführten Beatmungshüben, die Quadranten Q1 und Q2 für Asynchronie-Sequenzen aufgrund von zu spät ausgeführten Beatmungshüben. Im Quadranten Q4 wird die Asynchronie-Sequenz [0,1,3] (Reverse Triggering) veranschaulicht, im Quadranten Q1 die Asynchronie-Sequenz [0,2,3] (Late Triggering), im Quadranten Q3 die Asynchronie-Sequenz [3,2,0] (Premature Cycling Off) und im Quadranten Q2 die Asynchronie-Sequenz [3,1,0] (Late Cycling Off).

Im Beispiel von Figur 5 stehen die Quadranten Q3 und Q4 für Asynchronie-Sequenzen mit zusätzlichen, also fälschlichen Aktivitäten des Beatmungsgeräts 100. Diese fälschlichen Aktivitäten lassen sich als Extremfälle von "zu früh" bezeichnen. Im Quadranten Q1 wird die Asynchronie-Sequenz [0,2,0] (Missed Effort) veranschaulicht, im Quadranten Q2 die Asynchronie-Sequenz [3,1,3] (Missed Expiration), im Quadranten Q3 die Asynchronie-Sequenz [0,1,0] (Auto Triggering) und im Quadranten Q4 die Asynchronie-Sequenz [3,2,3] (Double Triggering). Bei diesen vier Asynchronie-Sequenzen hat das Beatmungsgerät 1 eine tatsächlich ausgeführte Einatmungs-Anstrengung des Patienten Pt nicht detektiert und daher auch keinen Beatmungshub ausgeführt oder fälschlich eine Einatmungs-Anstrengung detektiert und daraufhin einen Beatmungshub ausgeführt oder fälschlich nicht erkannt, dass der Patient Pt eine Einatmungs-Anstrengung beendet hat, oder fälschlich einen Beatmungshub beendet.

Sowohl im Beispiel von Figur 5 als auch im Beispiel von Figur 6 ist auf der x-Achse die mittlere Dauer der Asynchronie-Sequenzen der jeweiligen Art in [sec] oder der mittlere Anteil an der gesamten bisherigen Dauer der künstlichen Beatmung aufgetragen, z.B. in [%] seit dem Zeitpunkt, an dem die künstliche Beatmung dieses Patienten Pt begonnen wurde, oder seit der letzten Kalibrierung oder Justierung des Beatmungsgeräts 100, wobei die mittlere Dauer per arithmetischem Mittelwert oder per Median berechnet wird. Auf der y-Achse ist die Frequenz der Asynchronie-Sequenzen der jeweiligen Art in [Anzahl/min] oder als Anteil in [%] an den ausgeführten Beatmungshüben aufgetragen. Die Fläche für eine Art von Asynchronie-Sequenz ist umso größer, je länger und / oder häufiger diese Asynchronie-Sequenz insgesamt aufgetreten ist, und zeigt beispielsweise die gesamte Dauer der jeweiligen Asynchronie-Sequenz an. Möglich ist, die jeweilige Art einer Asynchronie-Sequenz durch eine Farbe oder durch eine sonstige von einem Menschen wahrnehmbare Codierung zu kennzeichnen. Möglich ist natürlich, dass mindestens eine Asynchronie-Sequenz überhaupt nicht auftritt und daher das entsprechende Rechteck fehlt.

In den beiden gezeigten Beispielen ist auf der x-Achse die gemittelte Dauer in [min] eingetragen, auf der y-Achse die Frequenz, also Anzahl pro Minute. Möglich ist auch, auf der x-Achse ein anderes Maß dafür, wie lange die Asynchron-Sequenzen der betreffenden Art aufgetreten sind, und auf der y-Achse ein anderes Maß dafür, wie gravierend eine Asynchronie-Sequenz die unterstützende künstliche Beatmung beeinträchtigt hat, zu verwenden.

In einer Ausgestaltung misst mindestens ein pneumatischer Sensor 2, 3, 7, 15 ein pneumatisches Maß für die eigene Atmungsaktivität des Patienten Pt und / oder die unterstützende künstliche Beatmung durch das Beatmungsgerät 100. Aus dem Signal dieses pneumatischen Sensors 2, 3, 7, 15 lassen sich die mechanische Anstrengung, die die eigene Atmungsmuskulatur des Patienten Pt aufbringt (Work of Breathing), oder der über die Zeit aufintegrierte Druck (Pressure-to-Product) herleiten. In einer Ausgestaltung wird auf der y-Achse für jede Asynchronie-Sequenz die jeweilige mechanische Atemanstrengung oder der aufintegrierte Druck aufgetragen.

In einer Ausgestaltung wird ein Gesamt-Rechteck Re um die maximal vier einzelnen Rechtecke für die vier Asynchronie-Sequenzen gelegt, vgl. Figur 5 und Figur 6. Das Gesamt-Rechteck Re umschließt alle diese vier einzelnen Rechtecke und ist so klein wie möglich. Die Größe des Gesamt-Rechtecks Re ist ein weiteres visuell wahrnehmbares Maß dafür, wie gut die Beatmungshübe mit der eigenen Atmungsaktivität synchronisiert sind. Diese Größe lässt sich rasch visuell wahrnehmen.

**Bezugszeichenliste**

| | |
|---|---|
| 2 | pneumatischer Atmungs-Sensor vor dem Mund des Patienten Pt, misst den Atemwegsdruck $P_{aw}$ und optional den Volumenstrom Vol', fungiert als der Atemwegsdruck-Sensor, umfasst die Bestandteile 2.1 und 2.2 |
| 2.1 | Messwertaufnehmer des Atmungs-Sensors 2, greift eine Gasprobe aus der Fluidverbindung zwischen der Lunge Lu des Patienten Pt und dem Beatmungsgerät 100 ab |
| 2.2 | eigentlicher Drucksensor des Atmungs-Sensors 2 |
| 3 | Sonde in der Speiseröhre Sp des Patienten Pt, misst den Speiseröhren-Druck $P_{es}$ und optional den gastralen Druck $P_{ga}$, mit dem Messkatheter 6 verbunden |
| 4 | Anschlussstück im Mund des Patienten Pt, mit dem Messkatheter 6 in der Speiseröhre Sp verbunden |
| 5.1.1, 5.1.2 | herznahes Paar von Messelektroden auf der Haut des Patienten Pt |
| 5.2.1, 5.2.2 | zwerchfellnahes Paar von Messelektroden auf der Haut des Patienten Pt |
| 6 | Messkatheter in der Speiseröhre Sp des Patienten Pt, mit der Messsonde 3 und dem Anschlussstück 4 verbunden |
| 7 | gastrale Sonde im Magen Ma des Patent Pt, misst den gastralen Druck $P_{ga}$ |
| 10 | signalverarbeitende Signalverarbeitungseinheit des Beatmungsgeräts 100, empfängt Messwerte von den Atmungs-Sensoren 2, 3, 5.1.1 bis 5.2.2, 7, generiert mindestens ein respiratorisches Signal $Sig_{res}$ und löst die Beatmungshübe des Beatmungsgeräts 100 aus |
| 11 | signalverarbeitende Überwachungseinheit für das Beatmungsgerät 100, überwacht, wie gut die Beatmungshübe des Beatmungsgeräts 100 mit der eigenen Atmungsaktivität des Patienten Pt synchronisiert sind, erzeugt eine Situationen-Abfolge, detektiert in der Situationen-Abfolge Asynchronie-Sequenzen und veranlasst, dass eine Darstellung der Asynchronie-Sequenzen auf der Anzeige- und Bedieneinheit 12 dargestellt wird |
| 12 | Anzeige- und Bedieneinheit des Beatmungsgeräts 100 |
| 15 | Sensor am Beatmungsgerät 100, misst den Volumenstrom Vol' |
| 100 | Beatmungsgerät, beatmet den Patienten Pt künstlich, umfasst die Anzeige- und Bedieneinheit 12, die Signalverarbeitungseinheit 10 und optional die Überwachungseinheit 11 |
| Atm(1), ..., Atm(4) | Atemzüge, die im Summen-Signal $Sig_{Sum}$ detektiert werden |
| Hz(x), Hz(y) | Herzschläge, die im Summen-Signal $Sig_{Sum}$ detektiert werden |
| Q1, ..., Q4 | Quadranten einer beispielhaften Darstellung, in der die jeweilige Häufigkeit und Dauer von vier Asynchronie-Sequenzen visualisiert werden |
| Re | Gesamt-Rechteck um die vier Rechtecke in den vier Quadranten Q1 bis Q4 |
| S.100 | schematischer zeitlicher Verlauf der Beatmungshübe, die das Beatmungsgerät 100 ausführt |
| S.Pt | schematischer zeitlicher Verlauf eines beispielhaften respiratorischen Signals, das mit der eigenen Atmungsaktivität des Patienten Pt korreliert |
| $Sig_{res}$ | pneumatisches respiratorisches Signal |
| $Sig_{sum}$ | elektrisches Summen-Signal, das aus einer Überlagerung eines kardiogenen Signals mit einem elektrischen respiratorischen Signal $Sig_{res}$ resultiert und aus Messwerten der Messelektroden 5.1.1 bis 5.2.2 erzeugt wird |

**Patentansprüche**

1. Beatmungs-Anordnung umfassend

   - ein Beatmungsgerät (100),
   - mindestens einen Atmungs-Sensor (3, 7, 15, 5.1.1, ..., 5.2.2) und
   - eine signalverarbeitende Überwachungseinheit (11),
   wobei der oder jeder Atmungs-Sensor (3, 7, 15, 5.1.1, ..., 5.2.2) dazu ausgestaltet ist, jeweils eine Größe zu messen, die mit den eigenen Einatmungs-Anstrengungen des Patienten (Pt) korreliert,
   wobei das Beatmungsgerät (100) dazu ausgestaltet ist, eine unterstützende künstliche Beatmung eines Patienten (Pt) durchzuführen und bei der unterstützenden künstlichen Beatmung

   - Messwerte von dem oder mindestens einem Atmungs-Sensor (3, 7, 15, 5.1.1, ..., 5.2.2) zu empfangen,
   - durch Auswertung von Messwerten des oder mindestens eines Atmungs-Sensors (3, 7, 15, 5.1.1, ..., 5.2.2) mindestens ein respiratorisches Signal ($Sig_{res}$) zu generieren, welches ein Maß für die eigenen Einatmungs-Anstrengungen des Patienten (Pt) ist, und
   - abhängig von dem oder mindestens einem respiratorischen Signal ($Sig_{res}$) eine Abfolge von Beatmungs-hüben mit dem Ziel durchzuführen,
   dass der jeweilige Beginn und das jeweilige Ende jeder Einatmungs-Anstrengung des Patienten (Pt) den Beginn bzw. das Ende genau eines Beatmungshubs auslösen,

   wobei mindestens zwei mögliche Asynchronie-Arten vorgegeben sind, die sich voneinander unterscheiden,
   wobei eine vorgegebene mögliche Asynchronie-Art eine Zeit-Asynchronie ist, die tatsächlich aufgetreten ist, wenn
   ein Beatmungshub früher oder später als die diesen Beatmungshub auslösende Einatmungs-Anstrengung beginnt oder endet,
   wobei eine vorgegebene mögliche Asynchronie-Art eine Ereignis-Asynchronie ist, die tatsächlich aufgetreten ist, wenn

   - ein Beatmungshub ohne eine Einatmungs-Anstrengung ausgelöst wird oder
   - eine Einatmungs-Anstrengung keinen Beatmungshub auslöst, und

   wobei die Überwachungseinheit (11) dazu ausgestaltet ist, automatisch

   - durch Auswertung des oder mindestens eines respiratorischen Signals ($Sig_{res}$) den jeweiligen Beginn und das jeweilige Ende jeder Einatmungs-Anstrengung des Patienten (Pt) zu detektieren,
   - den jeweiligen Beginn und das jeweilige Ende jedes Beatmungshubs zu ermitteln,
   - jedes tatsächliche Auftreten einer vorgegebenen möglichen Asynchronie-Art mindestens dann zu detektieren, wenn die Dauer dieses Auftretens oberhalb einer vorgegebenen Zeitdauer-Schranke liegt, und
   - jeweils ein Maß für die jeweilige Häufigkeit und die jeweilige Dauer des jeweiligen tatsächlichen Auftretens jeder vorgegebenen möglichen Asynchronie-Art während einer Abfolge von Beatmungshüben zu ermitteln,

   wobei die Überwachungseinheit (11) dazu ausgestaltet ist, automatisch mindestens eine Darstellung zu erzeugen und zu bewirken, dass die Darstellung in einer von einem Menschen wahrnehmbaren Form ausgegeben wird,
   wobei in dieser Darstellung unter Verwendung von zwei Achsen für mindestens zwei vorgegebene Asynchronie-Arten jeweils das Maß für die jeweilige Häufigkeit als auch das Maß für die jeweilige Dauer dieser Asynchronie-Art gezeigt wird,
   wobei in der Darstellung für jede dargestellte Asynchronie-Art jeweils eine zweidimensionale Fläche gezeigt wird und
   wobei die beiden Abmessungen dieser Fläche vom Maß für die Häufigkeit bzw. vom Maß für die Dauer abhängen.

2. Beatmungs-Anordnung nach Anspruch 1,
   **dadurch gekennzeichnet, dass**

   die Überwachungseinheit (11) ein Bestandteil des Beatmungsgeräts (100) ist und
   das Beatmungsgerät (100) ferner eine Signalverarbeitungseinheit (10) umfasst, die dazu ausgestaltet ist, automatisch

- Messwerte von dem oder mindestens einem Atmungs-Sensor (3, 7, 15, 5.1.1, ..., 5.2.2) zu empfangen,
- durch Auswertung von Messwerten des oder mindestens eines Atmungs-Sensors (3, 7, 15, 5.1.1, ..., 5.2.2) automatisch das oder mindestens ein respiratorisches Signal ($Sig_{res}$) zu generieren und
- die Abfolge von Beatmungshüben auszulösen.

3. Beatmungs-Anordnung nach Anspruch 1 oder Anspruch 2,
**dadurch gekennzeichnet, dass**

die Überwachungseinheit (11) dazu ausgestaltet ist, automatisch

zu jedem Abtast-Zeitpunkt einer vorgegebenen Abfolge von Abtast-Zeitpunkten zu ermitteln, welche der folgenden vier möglichen Situationen an diesem Abtast-Zeitpunkt vorliegt:

- der Patient (Pt) führt eine Einatmungs-Anstrengung aus, und das Beatmungsgerät (100) führt einen Beatmungshub aus,
- weder führt der Patient (Pt) eine Einatmungs-Anstrengung aus noch führt das Beatmungsgerät (100) einen Beatmungshub aus,
- der Patient (Pt) führt eine Einatmungs-Anstrengung aus, aber das Beatmungsgerät (100) führt keinen Beatmungshub aus,
- das Beatmungsgerät (100) führt einen Beatmungshub aus, aber der Patient (Pt) führt keine Einatmungs-Anstrengung aus, und

wobei die Überwachungseinheit (11) dazu ausgestaltet ist, ist, für die Ermittlung, welche Situation an einem Abtast-Zeitpunkt jeweils vorliegt, das oder mindestens ein respiratorisches Signal ($Sig_{res}$) auszuwerten,
wobei die Überwachungseinheit (11) dazu ausgestaltet ist, bei der Ermittlung der Maße für die Häufigkeit und / oder die Dauer der vorgegebenen möglichen Asynchronie-Arten

- eine Situationen-Abfolge zu ermitteln,
wobei die Situationen-Abfolge pro Abtast-Zeitpunkt der Abfolge die jeweils tatsächlich aufgetretene Situation umfasst,
- in dieser Situationen-Abfolge jede Situationen-Sequenz zu ermitteln,

wobei eine ermittelte Situationen-Sequenz aus mindestens zwei unterschiedlichen unmittelbar aufeinanderfolgenden Situationen besteht, die sich voneinander unterscheiden,
bevorzugt aus drei unmittelbar aufeinander folgenden Situationen besteht, und

- für jede vorgegebene mögliche Asynchronie-Art das Maß für deren Häufigkeit und / oder Dauer unter Verwendung derjenigen ermittelten Situationen-Sequenzen, bei denen diese Asynchronie-Art vorliegt, zu ermitteln.

4. Beatmungs-Anordnung nach einem der Ansprüche 1 bis 3,
**dadurch gekennzeichnet, dass**

das Beatmungsgerät (100) dazu ausgestaltet ist, auf das oder mindestens ein respiratorisches Signal ($Sig_{res}$) eine Entscheidungsvorschrift anzuwenden,
um automatisch zu entscheiden, wann der Patient (Pt) eine Einatmungs-Anstrengung beginnt und wann er sie beendet,
wobei diese Entscheidungsvorschrift von mindestens einem Parameter abhängt, und
die Überwachungseinheit (11) dazu ausgestaltet ist,

- mindestens einmal für den oder mindestens einen Parameter der Entscheidungsvorschrift einen Sollwert zu berechnen und
- zu bewirken, dass eine Nachricht erzeugt und an das Beatmungsgerät (100) übermittelt oder in einer von einem Menschen wahrnehmbaren Form ausgegeben wird,

wobei die Überwachungseinheit (11) weiterhin dazu ausgestaltet ist, den Sollwert abhängig von mindestens einem ermittelten Maß für die ermittelte Häufigkeit und / oder Dauer einer möglichen Asynchronie-Art zu berechnen,
wobei die Nachricht eine Information über den berechneten Sollwert und / oder über eine vom Sollwert abhängende Veränderung des aktuell verwendeten Werts des Parameters umfasst und
wobei das Beatmungsgerät (100) dazu ausgestaltet ist, automatisch die angewendete Entscheidungsvorschrift abhängig von der empfangenen Nachricht oder von einer Benutzereingabe zu verändern.

**Claims**

1.  Ventilation arrangement comprising

    - a ventilation device (100),
    - at least one respiration sensor (3, 7, 15, 5.1.1, ..., 5.2.2) and
    - a signal processing monitoring unit (11),
    wherein the or each respiration sensor (3, 7, 15, 5.1.1, ..., 5.2.2) is configured to measure a variable that correlates with the patient's (Pt) own inspiratory efforts,
    wherein the ventilation device (100) is configured to perform supportive artificial ventilation of a patient (Pt) and, during the supportive artificial ventilation,

    - to receive measured values from the or at least one respiration sensor (3, 7, 15, 5.1.1, ..., 5.2.2),
    - to generate at least one respiratory signal ($Sig_{res}$), which is a measure of the patient's (Pt) own inspiratory efforts, by evaluating measured values of the or at least one respiratory sensor (3, 7, 15, 5.1.1, ..., 5.2.2), and,
    - depending on the or at least one respiratory signal ($Sig_{res}$), to perform a sequence of ventilation strokes with the aim of

    each start and each end of every inspiratory effort by the patient (Pt) triggering the start and end of exactly one ventilation stroke,
    wherein at least two possible asynchrony types that differ from one another are predetermined,
    wherein a predetermined possible asynchrony type is a temporal asynchrony that has actually occurred when a ventilation stroke starts or ends earlier or later than the inspiratory effort that triggered said ventilation stroke,
    wherein a predetermined possible asynchrony type is an event asynchrony that has actually occurred when

    - a ventilation stroke is triggered without an inspiratory effort or
    - an inspiratory effort does not trigger a ventilation stroke, and

    wherein the monitoring unit (11) is configured to automatically

    - detect each start and each end of every inspiratory effort by the patient (Pt) by evaluating the or at least one respiratory signal ($Sig_{res}$),
    - determine each start and each end of every ventilation stroke,
    - detect every actual occurrence of a predetermined possible asynchrony type, at least if the duration of this occurrence is above a predetermined duration limit, and
    - determine a measure of the particular frequency and the particular duration of each actual occurrence of every predetermined possible asynchrony type during a sequence of ventilation strokes,

    wherein the monitoring unit (11) is configured to automatically generate at least one depiction and to cause the depiction to be output in a form perceivable by a human,
    wherein, in said depiction, using two axes for at least two predetermined asynchrony types, the measure for the particular frequency as well as the measure for the particular duration of said asynchrony type is shown,
    wherein, in the depiction, a two-dimensional surface is shown for each depicted asynchrony type and
    wherein the two dimensions of this surface depend on the measure of the frequency and the measure of the duration respectively.

2.  Ventilation arrangement according to claim 1,
    **characterized in that**

    the monitoring unit (11) is a component of the ventilation device (100) and
    the ventilation device (100) further comprises a signal processing unit (10) which is configured to automatically

    - receive measured values from the or at least one respiration sensor (3, 7, 15, 5.1.1, ..., 5.2.2),

- automatically generate the or at least one respiratory signal ($Sig_{res}$) by evaluating measured values of the or at least one respiratory sensor (3, 7, 15, 5.1.1, ..., 5.2.2) and
- trigger the sequence of ventilation strokes.

3. Ventilation arrangement according to either claim 1 or claim 2,
**characterized in that**

the monitoring unit (11) is configured to automatically

determine, at each sampling time point of a predetermined sequence of sampling time points, which of the following four possible situations is present at said sampling time point:

- the patient (Pt) makes an inspiratory effort, and the ventilation device (100) performs a ventilation stroke,
- neither the patient (Pt) makes an inspiratory effort nor does the ventilation device (100) perform a ventilation stroke,
- the patient (Pt) makes an inspiratory effort, but the ventilation device (100) does not perform a ventilation stroke,
- the ventilation device (100) performs a ventilation stroke, but the patient (Pt) does not make an inspiratory effort, and

wherein the monitoring unit (11) is configured to evaluate the or at least one respiratory signal ($Sig_{res}$) to determine in each case which situation is present at a particular sampling time point,
wherein, when determining the measures for the frequency and/or duration of the predetermined possible asynchrony types, the monitoring unit (11) is configured to

- determine a sequence of situations,

wherein the sequence of situations per sampling time point of the sequence comprises each situation that actually occurred,

- determine each sequence of situations in this sequence of situations,

wherein a determined sequence of situations consists of at least two different, immediately consecutive situations that differ from each other,
preferably of three immediately consecutive situations, and

- determine the measure of the frequency and/or duration for each predetermined possible asynchrony type using the identified sequences of situations in which this asynchrony type is present.

4. Ventilation arrangement according to any of claims 1 to 3,
**characterized in that**

the ventilation device (100) is configured to apply a decision rule to the or at least one respiratory signal ($Sig_{res}$), in order to automatically decide when the patient (Pt) starts and ends an inspiratory effort,
wherein this decision rule depends on at least one parameter, and
the monitoring unit (11) is configured to

- calculate a target value at least once for the or at least one parameter of the decision rule and
- cause a message to be generated and transmitted to the ventilation device (100) or output in a form perceivable by a human,

wherein the monitoring unit (11) is further configured to calculate the target value depending on at least one determined measure for the determined frequency and/or duration of a possible asynchrony type,
wherein the message comprises information about the calculated target value and/or about a change in the currently used value of the parameter depending on the target value, and
wherein the ventilation device (100) is configured to automatically change the applied decision rule depending on the received message or on a user input.

**Revendications**

1. Agencement respiratoire comprenant

   - un appareil d'assistance respiratoire (100),
   - au moins un capteur de respiration (3, 7, 15, 5.1.1, ..., 5.2.2) et
   - une unité de surveillance (11) traitant les signaux,
   dans lequel le capteur de respiration ou chaque capteur de respiration (3, 7, 15, 5.1.1, ..., 5.2.2) est configuré pour mesurer respectivement une grandeur qui est en corrélation avec les propres efforts d'inspiration du patient (Pt),
   dans lequel l'appareil d'assistance respiratoire (100) est configuré pour effectuer une respiration artificielle assistée d'un patient (Pt) et, lors de la respiration artificielle assistée,

   - recevoir des valeurs mesurées provenant du capteur de respiration ou d'au moins un capteur de respiration (3, 7, 15, 5.1.1, ..., 5.2.2),
   - générer, par évaluation de valeurs mesurées du capteur de respiration ou d'au moins un capteur de respiration (3, 7, 15, 5.1.1, ..., 5.2.2), au moins un signal respiratoire ($Sig_{res}$) qui est une mesure des propres efforts d'inspiration du patient (Pt), et
   - en fonction du signal respiratoire ou d'au moins un signal respiratoire ($Sig_{res}$), effectuer une série d'insufflations avec pour objectif

   que le début et la fin respectifs de chaque effort d'inspiration du patient (Pt) déclenchent le début ou la fin d'exactement une insufflation,
   dans lequel au moins deux types d'asynchronie possibles sont prédéfinis, lesquels diffèrent l'un de l'autre,
   dans lequel un type d'asynchronie possible prédéfini est une asynchronie temporelle qui s'est effectivement produite
   lorsqu'une insufflation commence ou se termine plus tôt ou plus tard que l'effort d'inspiration qui déclenche cette insufflation,
   dans lequel un type d'asynchronie possible prédéfini est une asynchronie d'événement qui s'est effectivement produite

   - lorsqu'une insufflation est déclenchée sans effort d'inspiration ou
   - lorsqu'un effort d'inspiration ne déclenche pas d'insufflation, et

   dans lequel l'unité de surveillance (11) est configurée pour

   - détecter automatiquement le début et la fin respectifs de chaque effort d'inspiration du patient (Pt) en évaluant le signal respiratoire ou au moins un signal respiratoire ($Sig_{res}$),
   - déterminer automatiquement le début et la fin respectifs de chaque insufflation,
   - détecter automatiquement chaque occurrence réelle d'un type d'asynchronie possible prédéfini au moins lorsque la durée de cette occurrence est supérieure à une limite de durée prédéfinie, et
   - déterminer automatiquement respectivement une mesure pour la fréquence respective et la durée respective de l'occurrence réelle respective de chaque type d'asynchronie possible prédéfini lors d'une série d'insufflations,

   dans lequel l'unité de surveillance (11) est configurée pour générer automatiquement au moins une représentation et pour faire en sorte que la représentation soit émise sous une forme perceptible par un être humain,
   dans lequel la mesure de la fréquence respective ainsi que la mesure de la durée respective de ce type d'asynchronie sont présentées dans cette représentation à l'aide de deux axes pour au moins deux types d'asynchronie prédéfinis,
   dans lequel une surface bidimensionnelle est présentée dans la représentation pour chaque type d'asynchronie représenté, et
   dans lequel les deux dimensions de cette surface dépendent respectivement de la mesure de la fréquence ou de la mesure de la durée.

2. Agencement respiratoire selon la revendication 1,
   **caractérisé en ce que**

   l'unité de surveillance (11) fait partie intégrante de l'appareil d'assistance respiratoire (100), et

l'appareil d'assistance respiratoire (100) comprend en outre une unité de traitement de signaux (10) qui est configurée pour

- recevoir des valeurs mesurées provenant du capteur de respiration ou d'au moins un capteur de respiration (3, 7, 15, 5.1.1, ..., 5.2.2),
- générer automatiquement le signal respiratoire ou au moins un signal respiratoire ($Sig_{res}$) en évaluant les valeurs mesurées du capteur de respiration ou d'au moins un capteur de respiration (3, 7, 15, 5.1.1, ..., 5.2.2) et
- déclencher automatiquement la série d'insufflations.

3. Agencement respiratoire selon la revendication 1 ou la revendication 2,
   **caractérisé en ce que**

   l'unité de surveillance (11) est configurée pour
   déterminer automatiquement, à chaque instant d'échantillonnage d'une série prédéfinie d'instants d'échantillonnage, laquelle des quatre situations possibles suivantes se présente à cet instant d'échantillonnage :

   - le patient (Pt) effectue un effort d'inspiration, et l'appareil d'assistance respiratoire (100) effectue une insufflation,
   - le patient (Pt) n'effectue pas d'effort d'inspiration, et l'appareil d'assistance respiratoire (100) n'effectue pas d'insufflation,
   - le patient (Pt) effectue un effort d'inspiration, mais l'appareil d'assistance respiratoire (100) n'effectue pas d'insufflation,
   - l'appareil d'assistance respiratoire (100) effectue une insufflation, mais le patient (Pt) n'effectue pas d'effort d'inspiration, et

   dans lequel l'unité de surveillance (11) est configurée pour évaluer le signal respiratoire ou au moins un signal respiratoire ($Sig_{res}$) afin de déterminer quelle situation se présente à un instant d'échantillonnage,
   dans lequel l'unité de surveillance (11) est configurée, lors de la détermination des mesures de la fréquence et/ou de la durée des types d'asynchronie possibles prédéfinis, pour

   - déterminer une série de situations

   dans lequel la série de situations comprend, pour chaque instant d'échantillonnage de la série, la situation qui s'est effectivement produite,

   - déterminer chaque séquence de situations dans cette série de situations,

   dans lequel une séquence de situations déterminée est constituée d'au moins deux situations différentes immédiatement consécutives qui sont différentes l'une de l'autre,
   est constituée de préférence de trois situations immédiatement consécutives, et

   - déterminer, pour chaque type d'asynchronie possible prédéfini, la mesure de sa fréquence et/ou de sa durée à l'aide des séquences de situations déterminées pour lesquelles ce type d'asynchronie est présent.

4. Agencement respiratoire selon l'une des revendications 1 à 3,
   **caractérisé en ce que**

   l'appareil d'assistance respiratoire (100) est configuré pour appliquer une règle de décision au signal respiratoire ou à au moins un signal respiratoire ($Sig_{res}$),
   pour décider automatiquement quand le patient (Pt) commence un effort d'inspiration et quand il le termine,
   dans lequel cette règle de décision dépend d'au moins un paramètre, et
   l'unité de surveillance (11) est configurée pour

   - calculer au moins une fois une valeur de consigne pour le paramètre ou au moins un paramètre de la règle de décision, et
   - faire en sorte qu'un message soit généré et transmis à l'appareil d'assistance respiratoire (100) ou émis sous une forme perceptible par un être humain,

dans lequel l'unité de surveillance (11) est en outre configurée pour calculer la valeur de consigne en fonction d'au moins une mesure déterminée pour la fréquence et/ou la durée déterminée d'un type d'asynchronie possible, dans lequel le message comprend une information sur la valeur de consigne calculée et/ou sur une modification, dépendante de la valeur de consigne, de la valeur actuellement utilisée du paramètre, et dans lequel l'appareil d'assistance respiratoire (100) est configuré pour modifier automatiquement la règle de décision appliquée en fonction du message reçu ou d'une entrée d'utilisateur.

EP 4 197 577 B1

FIG. 1

**FIG. 2a**

**FIG. 2b**

FIG. 3

EP 4 197 577 B1

FIG. 4

FIG. 5

EP 4 197 577 B1

FIG. 6

EP 4 197 577 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9392964 B2
- WO 2013071404 A1